# EUROPEAN PATENT SPECIFICATION

(11) **EP 1 255 512 B1**
(45) Date of publication and mention of the grant of the patent: **26.10.2005**
(21) Application number: 01904760.4
(22) Date of filing: 14.02.2001
(51) Int. Cl.: A61F 2/26

(54) **CONTROLLED PENILE PROSTHESIS**
KONTROLLIERBARE PENISPROTHESE
PROTHESE PENIENNE A COMMANDE

(30) Priority: 14.02.2000 US 182189 P; 14.02.2000 US 182205 P
(43) Date of publication of application: 13.11.2002
(73) Proprietor: Potencia Medical AG, 6341 Baar (CH)
(72) Inventor: FORSELL, Peter, CH-6313 Menzingen (CH)
(74) Representative: Höhfeld, Jochen
(86) International application number: PCT/SE2001/000309
(87) International publication number: WO 2001/047439

(56) References cited:
- EP-A1- 0 626 154
- US-A- 3 954 102
- US-A- 4 941 461
- US-A- 5 062 416

## Description

The present invention relates to a male sexual impotence treatment prosthesis apparatus, comprising an operable penile prosthesis implantable in the cavities of the corpora cavernosa of a patient's penile tissue to provide erect penile condition, when the prosthesis is operated, and a control device operable from outside the patient's body.

Male sexual impotence is a widespread problem. Many different solutions to this problem have been tried. In accordance with a prior system currently practised a hydraulic inflatable/contractible silicon prosthesis is implanted in the cavities of the corpora cavernosa of the penis. In fluid connection with this prosthesis is a reservoir implanted retroperitonially and a pump therefore in the scrotum. By manually pumping the pump the prosthesis is filled with fluid from the reservoir to achieve erect penile condition or is emptied of fluid, which returns to the reservoir, to achieve flaccid penile condition. However, there are several more or less severe disadvantages of this solution. A problem that often occurs is that thick, hard fibrosis is created around the pump which makes the system useless sooner or later.

Another solution to achieve erection is to restrict the blood flow leaving the penis. For example, U.S. Patent Nos. 4829990, 4958630 and 5048511 disclose two hydraulically operated inflatable cuffs wrapped around the respective crura or penile exit veins. A disadvantage of such a solution is that it involves complicated surgery. U.S. Patent No. 4828544 discloses another example on this solution, in which an artificial fistula system is surgically implanted and provides a primary fistula between the femoral artery and the femoral vein and a secondary fistula for leading blood from the primary fistula to the penis. An inflatable balloon engages the primary fistula between the secondary fistula and the vein. The balloon is in fluid connection with a manually compressible reservoir implanted in the scrotum. Again, implantation of this artificial fistula system requires delicate surgery.

Yet another solution is to inject a substance in the penile vein system to achieve erection. However, injections are painful and complicated for the patient.

Various impotence treatment devices in which fluid is distributed from a reservoir to an inflatable implanted prosthesis are disclosed in U.S. Pat. Nos. 3855122, 3954102, 4009711, 4201202, 4235227, 4318396 and 5250020.

U.S. Pat. No 4424807 discloses another solution in which inflatable hydraulic cylindrical elements are implanted relatively deep into the corpus cavernosum.

EP 0626154 discloses an implantable penile prosthesis system which includes an elongatable prosthesis that is operated by a remote control. A first fluid chamber is located adjacent to a distal end of the prosthesis that is inflatable with a pressurizing fluid, and a second fluid chamber is located adjacent to a proximal end of the prosthesis and stores the pressurizing fluid. An electromagnetic induction assembly serves to transfer fluid between the first and second chambers in response to operation of the remote control, so as to cause an erect condition in one mode and a flaccid condition in a second mode. The electromagnetic induction assembly is energised by an external, hand-held electromagnetic device or wand, which is powered by a battery.

US 4941461 discloses an electrically actuated, inflatable penile erection device. The device uses an alternating magnetic field generated from a wand external to the patient which is inductively coupled into a pickup coil that is implanted either within the corpus cavernosum of the penis or subcutaneously in the abdomen. The electrical energy from the pick-up coil is used to power an implanted pump that can pump saline solution from an implanted reservoir into the pendulous portion of the penile erection device. When the wand is placed over the base of the penis and turned on, a high intensity alternating current causes an alternating magnetic field to be impressed over the pick up coil. Alternating current that is produced in the coil is rectified and used to charge a capacitor which is discharged into a solenoid pump unit which causes fluid to be pumped to a chamber in the pendulous portion.

The object of the present invention to provide a simple male sexual impotence treatment prosthesis apparatus which is conveniently controlled by the patient.

This object is obtained by an apparatus of the kind described initially, which is characterised bya rechargeable battery implantable in the patient, and a control device operable from outside the patient's body for controlling the rechargeable battery to release energy for use in connection with the operation of the prosthesis, when the prosthesis is implanted.

As a result, the advantage is achieved that the prosthesis can be operated without need for touching subcutaneously implanted components of the apparatus. Furthermore, the apparatus of the invention provides simple and effective control of the energy supplied to implanted components of the apparatus which ensures an extended and reliable functionality of the apparatus, possibly for the rest of the patient's natural life, and at least many years.

Generally, the prosthesis is adapted to control the penis to change, preferably steplessly, between flaccid and erect penile condition. This gives the advantage that the patient is enabled to make fine adjustments of the prosthesis to achieve the desired erection without feeling pain.

The control device may also control the prosthesis. The control device may comprise an internal control unit, preferably including a microprocessor, implanted in the patient for controlling the prosthesis. The control device may further comprise an external control unit outside the patient's body, wherein the internal control unit is programmable by the external control unit, for example for controlling the prosthesis over a short period of time. Alternatively, the internal control unit may control the prosthesis over time in accordance with an activity schedule program, which may be adapted to the patient's needs. For example to avoid a too long duration of the penis in erect condition.

Conveniently, the external control unit may load the internal control unit with data in accordance with a loading mode only authorized for a doctor. For specialized controls of the implanted prosthesis, the external control unit may control the internal control unit in accordance with a doctor mode only authorized for the doctor. For simple controls of the implanted prosthesis, the external control unit may control the internal control unit in accordance with a patient mode permitted for the patient. Thus, by using the external control unit in accordance with different modes it is possible to have certain functions of the implanted prosthesis controlled by the patient and other more advanced functions controlled by the doctor, which enables a flexible post-operation treatment of the patient.

The apparatus of the invention may further comprise an external source of energy, wherein the control device controls the external source of energy to release wireless energy for use in connection with the operation of the prosthesis. The control device may be adapted to control the external source of energy to release energy, for instance to intermittently release energy in the form of a train of energy pulses, for direct use in connection with the operation of the prosthesis. In accordance with a suitable embodiment the control device controls the external source of energy to release electric energy, and the apparatus further comprises an implantable capacitor for producing the train of energy pulses from the released electric energy. In this case the term "*direct*" is used to mean, on one hand, that the released electric energy is used while it is being released by the control device, on the other hand, that the released electric energy may be somewhat delayed, in the order of seconds, by for instance an energy stabiliser before being used in connection with the operation of the prosthesis. The prosthesis may be operable in non-manual, a non-magnetic or non-mechanical manner by use of the released electric energy.

In accordance with a preferred embodiment of the invention, the apparatus comprises implantable electrical components including at least one, or only one single voltage level guard and a capacitor or accumulator, wherein the charge and discharge of the capacitor or accumulator is controlled by use of the voltage level guard. As a result, there is no need for any implanted current detector and/or charge level detector for the control of the capacitor, which makes the apparatus simple and reliable.

Generally, the apparatus further comprises an operation device implantable in the patient for operating the prosthesis, wherein the control device controls the operation device to operate the prosthesis. The control device may directly power the operation device with energy released from the external source of energy and/or power other energy consuming components of the apparatus to be implanted. In this case the term "*directly*" is used to mean, on one hand, that the operation device is powered with released energy while the latter is being released by the control device, on the other hand, that the released energy may be somewhat delayed, in the order of seconds, by for instance an energy stabiliser before powering the operation device. The advantage of directly using energy as it is released is that the apparatus can be of a very simple design and the few components involved makes the apparatus reliable.

The prosthesis may be non-inflatable, i.e. with no hydraulic fluid involved for the adjustments of the prosthesis. This eliminates problems with fluid leaking from the prosthesis.

The operation device may comprise hydraulic means and at least one valve for controlling a fluid flow in the hydraulic means. The control device may suitably comprise a wireless remote control for controlling the valve. The prosthesis may comprise hydraulic means and the operation device may comprise a reservoir forming a fluid chamber with a variable volume connected to the hydraulic means. The operation device may distribute fluid from the chamber to the hydraulic means by reduction of the volume of the chamber and withdraw fluid from the hydraulic means to the chamber by expansion of the volume of the chamber.

The rechargeable battery may store the wireless energy released from the external source of energy. Alternatively, a battery may be implanted in the patient for supplying electric energy to implanted electric energy consuming components of the apparatus, in addition to the supply of wireless energy. Where the control device comprises an implantable control unit the electronic circuit thereof and the prosthesis may be directly powered with transformed wireless energy, or energy from either the rechargeable battery or battery.

The wireless energy may be directly used for operation of the prosthesis, i.e. the prosthesis is operated as the wireless energy is being released from the external source of energy by the control device. In this case the term *"directly*" is used to mean, on one hand, that the prosthesis is promptly operated by using the released energy whithout first storing the latter, on the other hand, that the released energy may be somewhat delayed, in the order of seconds, by for instance an energy stabiliser before being used for the operation of the prosthesis. As a result, a very simple control of the prosthesis is achieved and there are only a few implanted components of the apparatus. For example, there is no implanted complicated signal control system. This gives the advantage that the apparatus will be extremely reliable.

Generally, the control device controls and directly or indirectly powers the operation device with wireless energy released from the external source of energy and/or powers other implanted energy consuming components of the apparatus.

In a first particular embodiment of the invention, the operation device comprises a motor, preferably an electric motor, which may have electrically conductive parts made of plastics. The motor may include a rotary motor, wherein the control device is adapted to control the rotary motor to rotate a desired number of revolutions. Alternatively, the motor may include a linear motor, or a hydraulic or pneumatic fluid motor, wherein the control device is adapted to control the fluid flow through the fluid motor. Motors currently available on the market are getting smaller and smaller. Furthermore, there is a great variety of control methods and miniaturized control equipment available. For example, a number of revolutions of a rotary motor may be analyzed by a Hall-element just a few mm in size.

In a second particular embodiment of the invention, the control device is adapted to shift polarity of the released energy to reverse the operation device. The operation device may suitably comprise an electric motor and the released energy may comprise electric energy.

In a third particular embodiment in accordance with the first and second main aspects of the invention there is a reversing device implantable in the patient for reversing the function performed by the prosthesis, i.e. to change from erect to flaccid penile condition, suitably in a stepless manner. In this connection, the control device suitably controls the .reversing device, which may include a switch, to reverse the function performed by the prosthesis. The reversing device may comprise hydraulic means including a valve for shifting the flow direction of a fluid in the hydraulic means. Alternatively, the reversing device may comprise a mechanical reversing device, such as a switch or a gearbox.

Where the reversing device comprises a switch the control device suitably controls the operation of the switch by shifting polarity of released energy supplied to the switch. The switch may comprise an electric switch and the source of energy may supply electric energy for the operation of the switch. The switch mentioned above may comprise an electronic switch or, where applicable, a mechanical switch.

In accordance with the third particular embodiment, the operation device preferably comprises a motor, wherein the reversing device reverses the motor.

In a fourth particular embodiment of the invention, the prosthesis comprises hydraulic means, for example including an expansible/contractible cavity for fluid. Preferably, the operation device is adapted to conduct hydraulic fluid in the hydraulic means, and comprises a motor, a valveless fluid conduit connected to the hydraulic means of the prosthesis, and a reservoir for fluid, wherein the reservoir forms part of the conduit. The operation device suitably comprises a pump operated by the motor. All of the hydraulic components involved are preferably deviod of any non-return valve. This is of great advantage, because with valves involved there is always a risk of malfunction due to inproperly working valves, especially when long time periods pass between valve operations. The reservoir may form a fluid chamber with a variable volume, and the pump may distribute fluid from the chamber to the hydraulic means of the prosthesis by reduction of the volume of the chamber and withdraw fluid from the hydraulic means to the chamber by expansion of the volume of the chamber.

The control device suitably controls the rechargeable battery from outside the patient's body to release electric energy. This solution is advantageous for embodiments of the apparatus that have a relatively high consumption of energy, which cannot be satisfied by direct supply of wireless energy.

The rechargeable battery preferably has a lifetime of at least 10 years.

The apparatus may further comprise a switch implanted in the patient for directly or indirectly switching the operation of the prosthesis, wherein the switch directly or indirectly affects the supply of energy from the rechargeable battery. This solution is advantageous for embodiments of the apparatus that have a relatively high energy consumption which cannot be met by direct supply of wireless energy.

In accordance with an embodiment of the invention, the switch switches between an off mode, in which the rechargeable battery is not in use, and an on mode, in which the rechargeable battery supplies energy for the operation of the prosthesis. In this case, the switch is conveniently operated by the wireless energy released from the external source of energy to switch between the on and off modes. The control device, preferably comprising a wireless remote control, may control the external source of energy to release the wireless energy. The advantage of this embodiment is that the lifetime of the implanted rechargeable battery can be significantly prolonged, since the implanted rechargeable battery does not supply energy when the switch is in its off mode.

In accordance with another embodiment of the invention, the control device comprises a wireless remote control for controlling the rechargeable battery. In this case, the switch is operable by the wireless energy from the external source of energy to switch between an off mode, in which the rechargeable battery and remote control are not in use, and a standby mode, in which the remote control is permitted to control the rechargeable battery to supply energy for the operation of the prosthesis.

In accordance with another embodiment of the invention, the apparatus further comprises an energy transforming device implanted in the patient for transforming the wireless energy into storable energy, wherein the rechargeable battery stores the storable energy. In this case, the switch switches from an off mode, in which the rechargeable battery is not in use, to an on mode, in which the rechargeable battery supplies energy for the operation of the prosthesis.

The control device, preferably comprising a wireless remote control, may control the switch to switch between the on and off modes.

All of the above embodiments may be combined with at least one implantable sensor for sensing at least one physical parameter of the patient, wherein the control device may control the prosthesis in response to signals from the sensor. For example, the sensor may directly or indirectly sense ejaculation or the pressure against the prosthesis or the pressure in the urethra or any other important physical parameter. The pressure sensor may be any suitable known or conventional pressure sensor such as shown in U.S. patents 5540731, 4846181, 4738267, 4571749, 4407296 or 3939823; or an NPC-102 Medical Angioplasty Sensor.

Advantageously, the sensor may sense ejaculation and the prosthesis may make the penis flaccid in response to the sensor sensing that ejaculation has occurred.

Where the control device comprises an internal control unit to be implanted in the patient, the internal control unit may suitably directly control the prosthesis in response to signals from the sensor. In response to signals from the sensor, for example pressure or any other important physical parameter, the internal control unit may send information thereon to outside the patient's body. The control unit may also automatically control the prosthesis in response to signals from the sensor, such as signals corresponding to ejaculation. For example, the control unit may control the prosthesis to make the penis flaccid in response to the sensor sensing an abnormally high pressure against the prosthesis.

Where the control device comprises an external control unit outside the patient's body, the external control unit may, suitably directly, control the prosthesis in response to signals from the sensor. The external control unit may store information on the physical parameter sensed by the sensor and may be manually operated to control the prosthesis based on the stored information. In addition, there may be at least one implantable sender for sending information on the physical parameter sensed by the sensor.

An external data communicator may be provided outside the patient's body and an internal data communicator to be implanted in the patient may be provided for communicating with the external data communicator. The internal data communicator may feed data related to the patient, or related to the prosthesis, back to the external data communicator. Alternatively or in combination, the external data communicator may feed data to the internal data communicator. The internal data communicator may suitably feed data related to at least one physical signal of the patient.

The apparatus may comprise an implantable energy transforming device, wherein the control device releases electric energy and the energy transforming device transforms the electric energy into kinetic energy for, preferably direct, operation of the prosthesis. Suitably, an implantable stabiliser, such as a capacitor or a rechargeable accumulator, or the like, may be provided for stabilising the electric energy released by the control device. In addition, the control device may control the external source of energy to release energy for a determined time period or in a determined number of energy pulses. Finally, the prosthesis may be non-inflatable.

All of the above embodiments are preferably remote controlled. Thus, the control device advantageously comprises a wireless remote control transmitting at least one wireless control signal for controlling the prosthesis. With such a remote control it will be possible to adapt the function of the apparatus to the patient's need.

The wireless remote control may be capable of obtaining information on the condition of the prosthesis and of controlling the prosthesis in response to the information. Also, The remote control may be capable of sending information related to the prosthesis from inside the patient's body to the outside thereof.

In a particular embodiment of the invention, the wireless remote control comprises at least one external signal transmitter or transceiver and at least one internal signal receiver or transceiver implantable in the patient. In another particular embodiment of the invention, the wireless remote control comprises at least one external signal reciever or transceiver and at least one internal signal transmitter or transceiver implantable in the patient.

The remote control may transmit a carrier signal for carrying the control signal, wherein the carrier signal is frequency, amplitude or frequency and amplitude modulated and is digital, analog or digital and analog. Also the control signal used with the carrier signal may be frequency, amplitude or frequency and amplitude modulated.

The control signal may comprise a wave signal, for example, a sound wave signal, such as an ultrasound wave signal, an electromagnetic wave signal, such as an infrared light signal, a visible light signal, an ultra violet light signal, a laser signal, a micro wave signal, a radio wave signal, an x-ray radiation signal, or a gamma radiation signal. Where applicable, two or more of the above signals may be combined.

The control signal may be digital or analog, and may comprise an electric or magnetic field. Suitably, the wireless remote control may transmit an electromagnetic carrier wave signal for carrying the digital or analog control signal. For example, use of an analog carrier wave signal carrying a digital control signal would give safe communication. The control signal may be transmitted in pulses by the wireless remote control.

The operation device preferably comprises an electrical operation device.

Typically the apparatus of the invention comprises an adjustment device for adjusting the prosthesis between erect and flaccid penile conditions. The adjustment device may be adapted to mechanically adjust the prosthesis. Alternatively, the adjustment device may be adapted to hydraulically adjust the prosthesis by using hydraulic means which is devoid of hydraulic fluid of the kind having a viscosity that substantially increases when exposed to heat or a magnetic field, *i.e.* the hydraulic fluid would not become more viscous when exposed to heat or influenced by magnetic forces.

The above-presented embodiments of the invention may be modified in accordance with the following suggestions. An implantable capacitor having a capacity less than 0,1 µF may be provided for producing the above-mentioned train of energy pulses.

An implantable motor or pump may be provided for operating the penile prosthesis, wherein the control device is adapted to control the external source of energy to directly power the motor or pump with the released energy. Specifically, the control device may be adapted to release wireless energy in the form of a magnetic field or electromagnetic waves (excluding radio waves) for direct power of the motor or pump, as the wireless energy is being released. Where a pump is used it preferably is not a plunger type of pump.

Generally, the wireless energy comprises a signal.

The apparatus may further comprise implantable energy transforming device for transforming wireless energy directly or indirectly into energy different than the wireless energy, for operation of the penile prosthesis. For example, the motor or pump may be powered by the transformed energy.

The energy transforming device may transform the wireless energy in the form of sound waves, preferably directly, into electric energy for operation of the penile prosthesis. The energy transforming device may comprise a capacitor adapted to produce electric pulses from the transformed electric energy.

The motor mentioned in the present specification may also be directly powered with wirelessly transmitted electromagnetic or magnetic energy in the form of signals, as the energy is transmitted. Furthermore, all the various functions of the motor and associated components described in the present specification may be used where applicable.

Generally, the penile prosthesis advantageously is embedded in a soft or gel-like material, such as a silicone material having hardness less than 20 Shore.

Of course, the penile prosthesis preferably is adjustable in a non-manual manner.

All the above described various components, such as the motor, pump and capacitor, may be combined in the different embodiments where applicable. Also the various functions described in connection with the above embodiments of the invention may be used in different applications, where applicable.

All the various ways of transferring energy and controlling the energy presented in the present specification may be practised by using all of the various components and solutions described.

There are also provided methods of treating an impotent male patient. These methods do not form part of the invention but are useful for understanding the invention. Accordingly, there is provided a method of treating an impotent male patient, comprising: (a) surgically implanting an operable prosthesis in the corpora cavernosa of the male patient. (b) Providing a source of energy. (c) Controlling the source of energy to release energy for use in connection with the operation of the prosthesis.

Steps (b) and (c) may further comprise providing the source of energy external to the patient's body and controlling the external source of energy from outside the patient's body to release wireless energy for use in connection with the operation of the prosthesis.

The method may further comprise (d) implanting in the patient an operation device which can adjust the prosthesis in response to supplied energy, and (f) using the released wireless energy to operate the implanted operation device to achieve erect or flaccid penile condition.

In accordance with an alternative method, there is provided a method of treating an impotent male patient, comprising the steps of placing at least two laparascopical trocars in the patient's body, inserting a tool through the trocars and using the tool to place an operable prosthesis in the cavities of the corpora cavernosa, providing a source of energy outside or inside the male patient's body, controlling the source of energy from outside the patient's body to release energy, which may comprise wireless energy where the source of energy is external to the patient's body, and using the released energy in connection with the operation of the prosthesis.

In accordance with another alternative method, there is provided a method of treating an impotent male patient, comprising the steps of placing at least two laparascopical trocars in the male patient's body, inserting a tool through the trocars and using the tool to place an operable prosthesis in the cavities of the corpora cavernosa, implanting an energy transforming device, providing an external source of energy, controlling the external source of energy to release wireless energy, and transforming the wireless energy by the energy transforming device into energy different than the wireless energy for use in connection with the operation of the prosthesis. This method may further comprise implanting a stabiliser in the patient's body to stabilize the energy transformed by the energy transforming device.

It is the primary object of the present invention to provide a simple yet effective apparatus for treating male sexual impotence. This and other objects of the invention will become clear from an inspection of the detailed description of the invention and from the appended claims.

The invention is described in more detail in the following with reference to the accompanying drawings, in which
FIGURES 1 to 6 are schematic block diagrams illustrating six embodiments, respectively, of the invention, in which wireless energy released from an external source of energy is used for direct operation of a prosthesis implanted in the penile tissue of a patient;
FIGURES 7 to 10 are schematic block diagrams illustrating four embodiments, respectively, of the invention, in which energy is released from an implanted source of energy;
FIGURES 11 to 15 are schematic block diagrams illustrating five embodiments, respectively, of the invention, in which a switch is implanted in the patient for directly or indirectly switching the operation of the prosthesis;
FIGURE 16 is a schematic block diagram illustrating conceivable combinations of implantable components for achieving various communication options;
FIGURE 17 illustrates the apparatus in accordance with the invention implanted in a patient;
FIGURE 18 is a block diagram illustrating remote control components of an embodiment of the invention; and
FIGURE 19 is a schematic view of exemplary circuitry used for the components of the block diagram of FIGURE 18.

Referring to the drawing figures, like reference numerals designate identical or corresponding elements throughout the several figures.

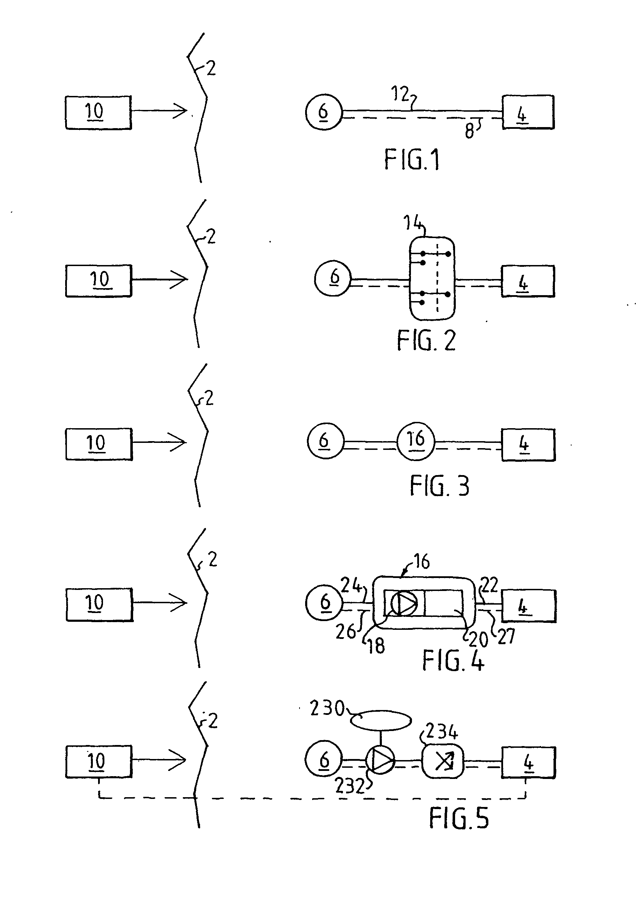

FIGURE 1 schematically shows an embodiment of the male sexual impotence treatment prosthesis apparatus of the invention having some parts implanted in a patient and other parts located outside the patient's body. Thus, in FIGURE 1 all parts placed to the right of the patient's skin 2 are implanted and all parts placed to the left of the skin 2 are located outside the patient's body.

The apparatus of FIGURE 1 comprises an implanted operable prosthesis 4, which is placed in the cavities of the corpora cavernosa of the patient's penis. The implanted prosthesis 4 is capable of performing a reversible function, i.e. to erect the penis or to make the penis flaccid. An implanted control unit 6 controls the implanted prosthesis 4 via a control line 8 to achieve an adequate erection. An external control unit 10 includes an external source of energy and a wireless remote control transmitting a control signal generated by the external source of energy. The control signal is received by a signal receiver incorporated in the implanted control unit 6, whereby the control unit 6 controls the implanted prosthesis 4 in response to the control signal. The implanted control unit 6 also uses energy from the control signal for directly operating the implanted prosthesis 4 via a power supply line 12, as the control signal is transmitted.

FIGURE 2 shows an embodiment of the invention identical to that of FIGURE 1, except that a reversing device in the form of a switch 14 also is implanted in the patient for reversing the implanted prosthesis 4. The control unit 6 uses the switch 14 to reverse the function performed by the implanted prosthesis 4. More precisely, the external control unit 10 releases energy carried by a wireless signal and the implanted control unit 6 transforms the wireless energy into a current for operating the switch 14. When the control unit 6 shifts the polarity of the current the switch 14 reverses the function performed by the implanted prosthesis 4.

FIGURE 3 shows an embodiment of the invention identical to that of FIGURE 1, except that an operation device in the form of a motor 16 also is implanted in the patient. The implanted control unit 6 powers the motor 16 with wireless energy released from the external source of energy of the external control unit 10. The implanted control unit 6 controls the operation of the motor 16 in response to a control signal from the remote control of the external control unit 10.

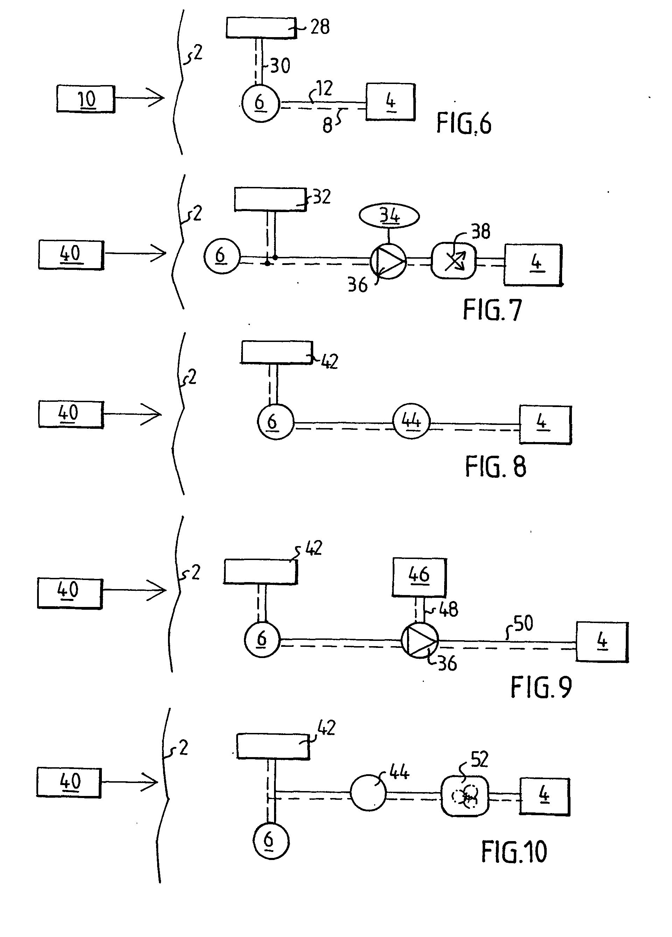

FIGURE 4 shows an embodiment of the invention identical to that of FIGURE 1, except that an assembly 16 including a motor/pump unit 18 and a fluid reservoir 20 also is implanted in the patient. In this case the prosthesis 4 is hydraulically operated, i.e. hydraulic fluid is pumped by the motor/pump unit 18 from the reservoir 20 through a conduit 22 to the prosthesis 4 to erect the penis, and hydraulic fluid is pumped by the motor/pump unit 18 back from the prosthesis 4 to the reservoir 20 to make the penis flaccid. The external control unit 10 releases energy carried by a wireless signal and the implanted control unit 6 transforms the wireless energy into a current, for example a current, for powering the motor/pump unit 18 via an electric power supply line 24. The implanted control unit 6 controls the motor/pump unit 16 and the prosthesis 4 via control lines 26 and 27.

FIGURE 5 shows an embodiment of the invention comprising the prosthesis 4, hydraulically operated, and the implanted control unit 6, and further comprising a hydraulic fluid reservoir 230, a motor/pump unit 232 and a reversing device in the form of a hydraulic valve shifting device 234, all of which are implanted in the patient. The motor of the motor/pump unit 232 is an electric motor.

FIGURE 6 shows an embodiment of the invention identical to that of FIGURE 1, except that an accumulator 28 also is implanted in the patient. The control unit 6 stores energy received from the external control unit 10 in the accumulator 28. In response to a control signal from the external control unit 10 the implanted control unit 6 releases energy from the accumulator 28 via a power line 30 for the operation of the prosthesis 4.

FIGURE 7 shows an embodiment of the invention comprising the prosthesis 4, hydraulically operated, and the implanted control unit 6, and further comprising a source of energy in the form of a battery 32, a hydraulic fluid reservoir 34, a motor/pump unit 36 and a reversing device in the form of a hydraulic valve shifting device 38, all of which are implanted in the patient. The motor of the motor/pump unit 36 is an electric motor. An external control unit 40 includes a wireless remote control transmitting a control signal which is received by the signal receiver incorporated in the implanted control unit 6.

In response to a control signal from the external control unit 40 the implanted control unit 6 powers the motor/pump unit 36 with energy from the battery 32, whereby the motor/pump unit 36 distributes hydraulic fluid between the reservoir 34 and the prosthesis 4. The control unit 6 controls the shifting device 38 to shift the hydraulic fluid flow direction between one direction in which the fluid is pumped by the motor/pump unit 36 from the reservoir 34 to the prosthesis 4 to erect the penis, and another opposite direction in which the fluid is pumped by the motor/pump unit 36 back from the prosthesis 4 to the reservoir 34 to make the penis flaccid.

FIGURE 8 shows an embodiment of the invention identical to that of FIGURE 6, except that a battery 42 is substituted for the accumulator 28, the external control unit 40 of the embodiment of FIGURE 5 is substituted for the external control unit 10 and an electric motor 44 is implanted in the patient for operating the prosthesis 4. In response to a control signal from the external control unit 40 the implanted control unit 6 powers the motor 44 with energy from the battery 42, whereby the motor 44 operates the prosthesis 4.

FIGURE 9 shows an embodiment of the invention identical to that of FIGURE 8, except that the motor/pump unit 36 of the embodiment of FIGURE 7 is substituted for the motor 44 and a fluid reservoir 46 also is implanted in the patient. The reservoir 46 is via fluid conduits 48 and 50 connected to the motor/pump unit 36 and prosthesis 4, which in this case is hydraulically operated. In response to a control signal from the external control unit 40, the implanted control unit 6 powers the electric motor of the motor/pump unit 36 with energy from the battery 42, whereby the motor/pump unit 36 distributes hydraulic fluid between the fluid reservoir 46 and the prosthesis 4.

FIGURE 10 shows an embodiment of the invention identical to that of FIGURE 8, except that a mechanical reversing device in the form of a gearbox 52 also is implanted in the patient. The implanted control unit 6 controls the gearbox 52 to reverse the function performed by the prosthesis 4 (mechanically operated).

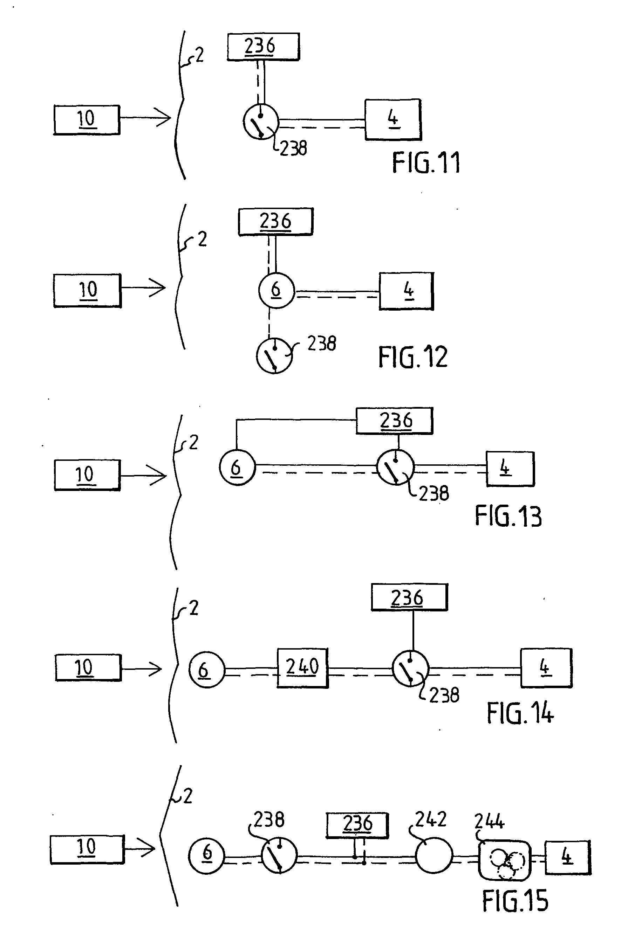

FIGURE 11 shows an embodiment of the invention comprising the prosthesis 4, the external control unit 10, an implanted source of energy 236 and an implanted switch 238. The switch 238 is operated by wireless energy released from the external source of energy of the external control unit 6 to switch between an off mode, in which the implanted source of energy 236 is not in use, and an on mode, in which the implanted source of energy 236 supplies energy for the operation of the prosthesis 4.

FIGURE 12 shows an embodiment of the invention identical to that of FIGURE 11, except that also the control unit 6 is implanted, in order to receive a control signal from the wireless remote control of the external control unit 10. The switch 238 is operated by the wireless energy from the external source of energy 10 to switch between an off mode, in which the implanted source of energy 236 and the wireless remote control of the external control unit 10 are not in use, *i.e.* the control unit 6 is not capable of receiving the control signal, and a standby mode, in which the wireless remote control is permitted to control the internal source of energy 236, via the implanted control unit 6, to supply energy for the operation of the prosthesis 4.

FIGURE 13 shows an embodiment of the invention identical to that of FIGURE 12, except that an energy transforming device for transforming the wireless energy into storable energy is incorporated in the implanted control unit 6 and that the implanted source of energy 236 is of a type that is capable of storing the storable energy. In this case, in response to a control signal from the external control unit 10, the implanted control unit 6 controls the switch 238 to switch from an off mode, in which the implanted source of energy 236 is not in use, to an on mode, in which the source of energy 36 supplies energy for the operation of the prosthesis 4.

FIGURE 14 shows an embodiment of the invention identical to that of FIGURE 13, except that an energy storage device 240 also is implanted in the patient for storing the storable energy transformed from the wireless energy by the transforming device of the control unit 6. In this case, the implanted ontrol unit 6 controls the energy storage device 240 to operate the switch 238 to switch between an off mode, in which the implanted source of energy 236 is not in use, and an on mode, in which the implanted source of energy 236 supplies energy for the operation of the prosthesis 4.

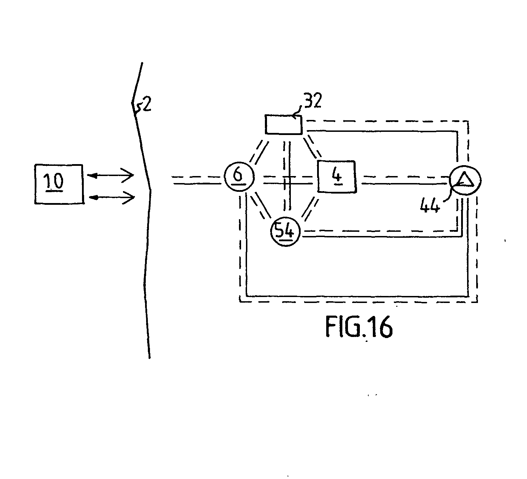

FIGURE 15 shows an embodiment of the invention identical to that of FIGURE 13, except that a motor 242 and a mechanical reversing device in the form of a gearbox 244 also are implanted in the patient. The implanted control unit 6 controls the gearbox 244 to reverse the function performed by the prosthesis 4 (mechanically operated), i.e. erecting the penis and making the penis flaccid.

FIGURE 16 schematically shows conceivable combinations of implanted components of the apparatus for achieving various communication possibilities. Basically, there are the implanted prosthesis 4, the implanted control unit 6 and the external control unit 10 including the external source of energy and the wireless remote control. As already described above the remote control transmits a control signal generated by the external source of energy, and the control signal is received by a signal receiver incorporated in the implanted control unit 6, whereby the control unit 6 controls the implanted prosthesis 4 in response to the control signal.

A sensor 54 may be implanted in the patient for sensing a physical parameter of the patient, such as the pressure in the stomach. The control unit 6, or alternatively the external control unit 10, may control the prosthesis 4 in response to signals from the sensor 54. A transceiver may be combined with the sensor 54 for sending information on the sensed physical parameter to the external control unit 10. The wireless remote control of the external control unit 10 may comprise a signal transmitter or transceiver and the implanted control unit 6 may comprise a signal receiver or transceiver. Alternatively, the wireless remote control of the external control unit 10 may comprise a signal receiver or transceiver and the implanted control unit 6 may comprise a signal transmitter or transceiver. The above transceivers, transmitters and receivers may be used for sending information or data related to the prosthesis from inside the patient's body to the outside thereof.

The motor 44 may be implanted for operating the prosthesis 4 and also the battery 32 may be implanted for powering the motor 44. The battery 32 may be equipped with a transceiver for sending information on the charge condition of the battery.

Those skilled in the art will realize that the above various embodiments according to FIGURES 1-15 could be combined in many different ways. For example, the energy operated switch 14 could be incorporated in any of the embodiments of FIGURES 4,6,8-10. The hydraulic shifting device 38 could be incorporated in any of the embodiments of FIGURES 4 and 9. The gearbox 52 could be incorporated in any of the embodiments of FIGURES 1,6 and 8.

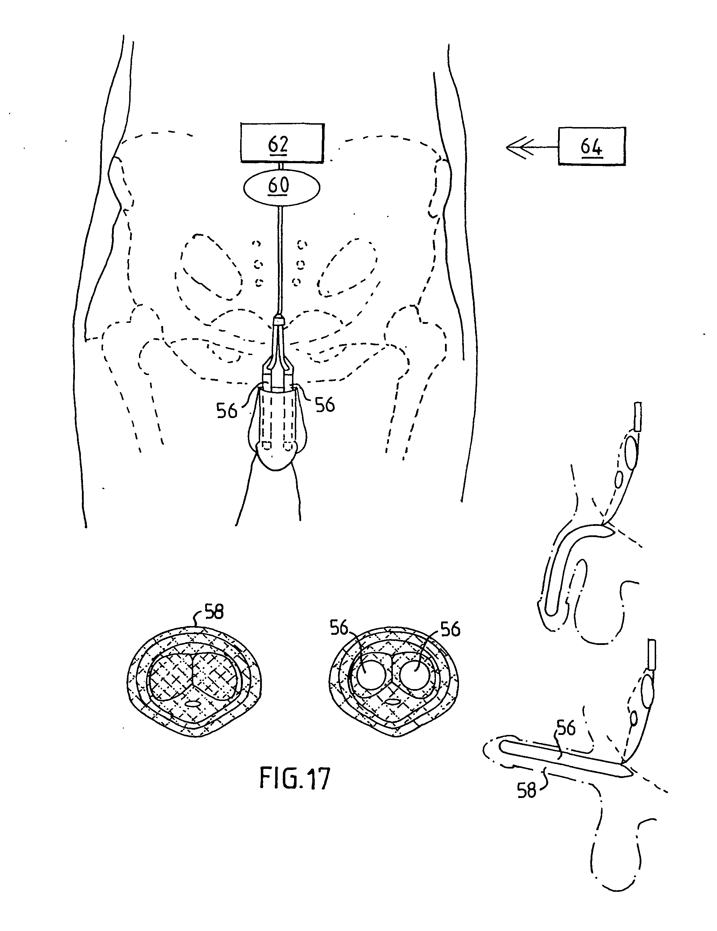

FIGURE 17 illustrates how any of the above-described embodiments of the impotence treatment prosthesis apparatus of the invention may be implanted in a patient. Thus, the apparatus comprises a prosthesis including two elongated balloon elements 56 implanted in the cavities of the corpora cavernosa of the patient's penile tissue 58, and an implanted hydraulic operation device 60 for operating the prosthesis elements 56. A control device in the form of a wireless remote control comprises an implanted control unit 62, which includes a signal receiver, for controlling the operation device 60, and an external control unit 64 including a signal transmitter for transmitting a control signal to the signal receiver of the implanted control unit 62. The implanted control unit 62 is capable of transforming signal energy from the control signal into electric energy for powering the operation device 60 and for energizing electric energy consuming implanted components of the apparatus. When the patient desires to achieve erection, he uses the external control unit 64 to activate the operation device 60 to distribute hydraulic fluid into the two balloon elements 56, whereby each element 56 is inflated and assumes the shape of a straight rod as indicated in FIGURE 17.

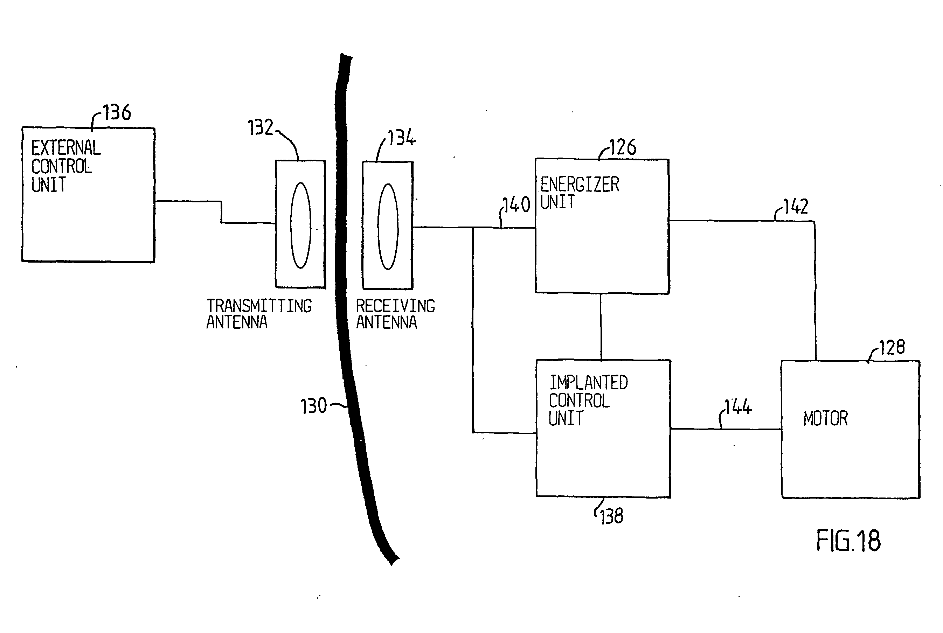

FIGURE 18 shows the basic parts of a wireless remote control of the apparatus of the invention including an electric motor 128 for operating a prosthesis, for example of the type illustrated in FIGURE 17. In this case, the remote control is based on the transmission of electromagnetic wave signals, often of high frequencies in the order of 100 kHz - 1 gHz, through the skin 130 of the patient. In FIGURE 18, all parts placed to the left of the skin 130 are located outside the patient's body and all parts placed to the right of the skin 130 are implanted. Any suitable remote control system may be used.

An external signal transmitting antenna 132 is to be positioned close to a signal receiving antenna 134 implanted close to the skin 130. As an alternative, the receiving antenna 134 may be placed for example inside the abdomen of the patient. The receiving antenna 134 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 132 comprises a coil having about the same restriction as the coil of the receiving antenna 134 but wound with a thick wire that can handle the larger currents that is necessary. The coil of the transmitting antenna 132 is tuned to the same specific high frequency as the coil of the receiving antenna 134.

An external control unit 136 comprises a microprocessor, a high frequency electromagnetic wave signal generator and a power amplifier. The microprocessor of the control unit 136 is adapted to switch the generator on/off and to modulate signals generated by the generator to send digital information via the power amplifier and the antennas 132,134 to an implanted control unit 138. To avoid that accidental random high frequency fields trigger control commands, digital signal codes are used. A conventional keypad placed on the external control unit 136 is connected to the microprocessor thereof. The keypad is used to order the microprocessor to send digital signals to activate the prosthesis to either restrict or enlarge the blood flow passageway. The microprocessor starts a command by applying a high frequency signal on the antenna 132. After a short time, when the signal has energized the implanted parts of the control system, commands are sent to restrict or enlarge the blood flow passageway in predefined steps. The commands are sent as digital packets in the form illustrated below.

| | | | |
|---|---|---|---|
| Start pattern, 8 bits | Command, 8 bits | Count, 8 bits | Checksum, 8 bits |

The commands are sent continuously during a rather long time period (*e.g.* about 30 seconds or more). When a new restrict or enlarge step is desired the Count byte is increased by one to allow the implanted control unit 138 to decode and understand that another step is demanded by the external control unit 136. If any part of the digital packet is erroneous, its content is simply ignored.

Through a line 140, an implanted energizer unit 126 draws energy from the high frequency electromagnetic wave signals received by the receiving antenna 134. The energizer unit 126 stores the energy in a power supply, such as a large capacitor, powers the control unit 138 and powers the electric motor 128 via a line 142.

The control unit 138 comprises a demodulator and a microprocessor. The demodulator demodulates digital signals sent from the external control unit 136. The microprocessor of the control unit 138 receives the digital packet, decodes it and, provided that the power supply of the energizer unit 126 has sufficient energy stored, sends a signal via a signal line 144 to the motor 128 to operate the prosthesis to either erect the penis or make the penis flaccid depending on the received command code.

Alternatively, the energy stored in the power supply of the energizer unit may only be used for powering a switch, and the energy for powering the motor 128 may be obtained from another implanted power source of relatively high capacity, for example a battery. In this case the switch is adapted to connect the battery to the control unit 138 in an on mode when the switch is powered by the power supply and to keep the battery disconnected from the control unit in a standby mode when the switch is unpowered.

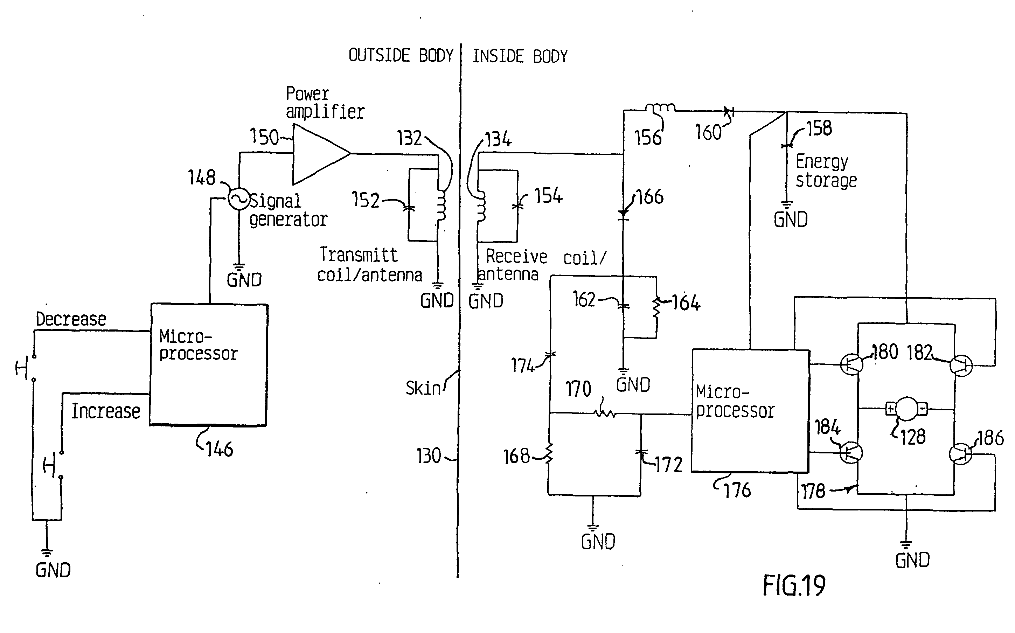

With reference to FIGURE 19, the remote control schematically described above will now be described in accordance with a more detailed embodiment. The external control unit 136 comprises a microprocessor 146, a signal generator 148 and a power amplifier 150 connected thereto. The microprocessor 146 is adapted to switch the signal generator 148 on/off and to modulate signals generated by the signal generator 148 with digital commands that are sent to implanted components of the apparatus. The power amplifier 150 amplifies the signals and sends them to the external signal transmitting antenna 132. The antenna 132 is connected in parallel with a capacitor 152 to form a resonant circuit tuned to the frequency generated by the signal generator 148.

The implanted signal receiving antenna coil 134 forms together with a capacitor 154 a resonant circuit that is tuned to the same frequency as the transmitting antenna 132. The signal receiving antenna coil 134 induces a current from the received high frequency electromagnetic waves and a rectifying diode 160 rectifies the induced current, which charges a storage capacitor 158. A coil 156 connected between the antenna coil 134 and the diode 160 prevents the capacitor 158 and the diode 160 from loading the circuit of the signal receiving antenna 134 at higher frequencies. Thus, the coil 156 makes it possible to charge the capacitor 158 and to transmit digital information using amplitude modulation.

A capacitor 162 and a resistor 164 connected in parallel and a diode 166 forms a detector used to detect amplitude modulated digital information. A filter circuit is formed by a resistor 168 connected in series with a resistor 170 connected in series with a capacitor 172 connected in series with the resistor 168 via ground, and a capacitor 174, one terminal of which is connected between the resistors 168,170 and the other terminal of which is connected between the diode 166 and the circuit formed by the capacitor 162 and resistor 164. The filter circuit is used to filter out undesired low and high frequencies. The detected and filtered signals are fed to an implanted microprocessor 176 that decodes the digital information and controls the motor 128 via an H-bridge 178 comprising transistors 180,182,184 and 186. The motor 128 can be driven in two opposite directions by the H-bridge 178.

The microprocessor 176 also monitors the amount of stored energy in the storage capacitor 158. Before sending signals to activate the motor 128, the microprocessor 176 checks whether the energy stored in the storage capacitor 158 is enough. If the stored energy is not enough to perform the requested operation, the microprocessor 176 waits for the received signals to charge the storage capacitor 158 before activating the motor 128.

## Claims

1. A male sexual impotence treatment prosthesis apparatus, comprising an operable penile prosthesis (4) implantable in the cavities of the corpora cavernosa of a patient to provide erect penile condition, when the prosthesis is operated, and a control device (6) operable from outside the patient's body, **characterized by** a rechargeable battery implantable in the patient, the control device controlling the rechargeable battery (32) to release energy for use in connection with the operation of the prosthesis, when the prosthesis is implanted.

2. An apparatus according to claim 1, further comprising an external source of energy, wherein the control device is adapted to control the external source of energy to release wireless energy for use in connection with the operation of the prosthesis.

3. An apparatus according to claim 1 or 2, wherein the control device controls the prosthesis.

4. An apparatus according to claim 3, wherein the control device comprises an internal control unit implantable in the patient for controlling the prosthesis.

5. An apparatus according to claim 4, wherein the internal control unit is programmable.

6. An apparatus according to claim 5, wherein the control device comprises an external control unit intended to be outside the patient's body, the internal control unit being programmable by the external control unit.

7. An apparatus according to claim 5, wherein the internal control unit is programmable for controlling the prosthesis over time.

8. An apparatus according to claim 7, wherein the internal control unit controls the prosthesis over time in accordance with an activity schedule program.

9. An apparatus according to claim 7, wherein the internal control unit comprises a microprocessor.

10. An apparatus according to claim 6, wherein the external control unit loads the internal control unit with data in accordance with a loading mode only authorized for a doctor.

11. An apparatus according to claim 6, wherein the external control unit controls the internal control unit in accordance with a doctor mode only authorized for a doctor.

12. An apparatus according to claim 6, wherein the external control unit controls the internal control unit in accordance with a patient mode permitted for the patient.

13. An apparatus according to claim 1, wherein the rechargeable battery has a lifetime of at least 10 years.

14. An apparatus according to any of claims 1-13, wherein the control device controls the penile prosthesis.

15. An apparatus according to claim 1 or 2, further comprising a battery implantable in the patient for supplying electric energy to implantable electric energy consuming components of the apparatus.

16. The apparatus according to claim 2, wherein the control device is adapted to control the external source of energy to release wireless energy for direct use in connection with the operation of the penile prosthesis.

17. The apparatus according to claim 16, wherein the control device is adapted to control the external source of energy to intermittently release wireless energy in the form of a train of energy pulses for direct use in connection with the operation of the penile prosthesis.

18. The apparatus according to any of claims 1,2 and 14, further comprising a switch implantable in the patient for directly or indirectly switching the operation of the penile prosthesis.

19. The apparatus according to claim 18, wherein the switch directly or indirectly affects the supply of energy from the rechargeable battery.

20. The apparatus according to claim 19, wherein the switch switches between an "off" mode, in which the rechargeable battery is not in use, and an "on" mode, in which the rechargeable battery supplies energy for the operation of the penile prosthesis.

21. The apparatus according to claim 2 and 20, wherein the switch is operable by the wireless energy released from the external source of energy.

22. The apparatus according to claim 21, wherein the control device controls the external source of energy to release the wireless energy.

23. The apparatus according to any of claims 1,2 and 22, wherein the control device comprises a wireless remote control.

24. The apparatus according to claim 19, wherein the control device comprises a wireless remote control for controlling the rechargeable battery.

25. The apparatus according to claim 24, wherein the switch is operable by the wireless energy from the external source of energy to switch between an "off" mode, in which the rechargeable battery and remote control are not in use, and a "standby" mode, in which the remote control is permitted to control the rechargeable battery to supply energy for the operation of the penile prosthesis.

26. The apparatus according to claim 14, wherein the control device comprises a wireless remote control.

27. The apparatus according to claim 19, further comprising an energy transforming device implantable in the patient for transforming the wireless energy into storable energy, wherein the rechargeable battery is capable of storing the storable energy.

28. The apparatus according to claim 27, wherein the switch switches from an "off" mode, in which the rechargeable battery is not in use, to an "on" mode, in which the rechargeable battery supplies energy for the operation of the penile prosthesis.

29. The apparatus according to claim 28, wherein the control device controls the switch to switch between the "on" and "off" modes.

30. The apparatus according to claim 29, wherein the control device comprises a wireless remote control.

31. An apparatus according to any one of claims 1,2 or 18, further comprising an operation device implantable in the patient for operating the penile prosthesis.

32. An apparatus according to claim 31, wherein the control device controls the operation device to operate the penile prosthesis.

33. An apparatus according to claim 32, wherein the operation device comprises hydraulic means and at least one valve for controlling a fluid flow in the hydraulic means.

34. An apparatus according to claim 33, wherein the control device comprises a wireless remote control for controlling the valve.

35. An apparatus according to claim 32, wherein the penile prosthesis comprises hydraulic means and the operation device comprises a reservoir forming a fluid chamber with a variable volume connected to the hydraulic means, and the operation device is adapted to distribute fluid from the chamber to the hydraulic means by reduction of the volume of the chamber and to withdraw fluid from the hydraulic means to the chamber by expansion of the volume of the chamber.

36. An apparatus according to claim 31 or 32, wherein the operation device comprises a motor.

37. An apparatus according to claim 36, wherein the motor comprises a rotary motor, and the control device controls the rotary motor to rotate a desired number of revolutions.

38. An apparatus according to claim 36, wherein the motor comprises a linear motor.

39. An apparatus according to claim 36, wherein the motor comprises a hydraulic or pneumatic fluid motor, and the control device controls the fluid motor.

40. An apparatus according to claim 36, wherein the motor comprises an electric motor having electrically conductive parts made of plastics.

41. An apparatus according to any one of claims 1,31 and 32, wherein the control device releases polarized energy from the source of energy.

42. An apparatus according to claim 31 or 32, wherein the control device shifts polarity of the released energy to reverse the operation device.

43. An apparatus according to claim 31 or 32, wherein the operation device comprises an electric motor and the released energy comprises electric energy.

44. An apparatus according to any one of claims 1, 31, 32 and 36, wherein the penile prosthesis is operable to perform a reversible function.

45. An apparatus according to claim 44, further comprising a reversing device implantable in the patient for reversing the function performed by the penile prosthesis.

46. An apparatus according to claim 45, wherein the control device controls the reversing device to reverse the function performed by the penile prosthesis.

47. An apparatus according to claim 45, wherein the reversing device comprises hydraulic means including a valve for shifting the flow direction of a fluid in the hydraulic means.

48. An apparatus according to claim 45, wherein the reversing device comprises a mechanical reversing device.

49. An apparatus according to claim 45, wherein the mechanical reversing device comprises a switch.

50. An apparatus according to claim 45, wherein the reversing device comprises a gearbox.

51. An apparatus according to claim 45, wherein the reversing device comprises a switch.

52. An apparatus according to claim 51, wherein the switch of the reversing device is operable by the released energy.

53. An apparatus according to claim 52, wherein the control device controls the operation of the switch of the reversing device by shifting polarity of the released energy supplied to the switch.

54. An apparatus according to claim 51, wherein the switch comprises an electric switch and the rechargeable battery supplies electric energy for the operation of the switch.

55. An apparatus according to claim 51, wherein the operation device comprises a motor, and the reversing device reverses the motor.

56. An apparatus according to any of claims 1,2, 31 and 32, wherein the penile prosthesis comprises hydraulic means, further comprising an operation device adapted to conduct a hydraulic fluid in the hydraulic means.

57. An apparatus according to claim 56, wherein the operation device comprises a motor.

58. An apparatus according to claim 56 or 57, wherein the operation device comprises a fluid conduit connected to the hydraulic means of the penile prosthesis, and a reservoir for fluid, the reservoir forming part of the conduit.

59. An apparatus according to claim 58, wherein the hydraulic means and conduit are devoid of any non-return valve.

60. An apparatus according to claim 59, wherein the reservoir forms a fluid chamber with a variable volume, and the operation device is adapted to distribute fluid from the chamber to the hydraulic means of the penile prosthesis by reduction of the volume of the chamber and to withdraw fluid from the hydraulic means to the chamber by expansion of the volume of the chamber.

61. An apparatus according to any of the preceding claims, further comprising at least one implantable sensor for sensing at least one physical parameter of the patient.

62. An apparatus according to claim 61, wherein the sensor is adapted to directly or indirectly sense as the physical parameter ejaculation or the pressure in the urethra.

63. An apparatus according to claim 61 or 62, wherein the control device controls the penile prosthesis in response to signals from the sensor.

64. An apparatus according to claim 63, wherein the control device comprises an internal control unit implantable in the patient, the internal control unit controlling the penile prosthesis in response to signals from the sensor.

65. An apparatus according to claim 64, wherein the control device comprises an external control unit outside the patient's body, the external control unit controlling the penile prosthesis in response to signals from the sensor.

66. An apparatus according to claim 65, wherein the external control unit stores information on the physical parameter sensed by the sensor and is manually operated to control the penile prosthesis based on the stored information.

67. An apparatus according to any of claims 61-66, further comprising at least one implantable sender for sending information on the physical parameter sensed by the sensor.

68. An apparatus according to any of the preceding claims, further comprising an external data communicator intended to be outside the patient's body and an internal data communicator implantable in the patient for communicating with the external communicator, wherein the internal data communicator feeds data related to the patient back to the external data communicator or the external data communicator feeds data to the internal data communicator.

69. An apparatus according to claim 68, wherein the internal data communicator feeds data related to the penile prosthesis.

70. An apparatus according to claim 68 or 69, wherein the implantable communicator feeds data related to at least one physical signal of the patient.

71. An apparatus according to any of the preceding claims, wherein the prosthesis is adapted to control the penis to change between flaccid and erect penile condition.

72. An apparatus according to claim 71, wherein the prosthesis is operable to erect the penis or to make it flaccid.

73. An apparatus according to claim 71 or 72, wherein the prosthesis is adapted to control the penis to steplessly change between flaccid and erect penile condition.

74. An apparatus according to any of the preceding claims, further comprising an external source of energy and an implantable energy transforming device, wherein the control device is adapted to control the external source of energy to release wireless electric energy and the energy transforming device is adapted to transform the electric energy into kinetic energy for operation of the prosthesis.

75. An apparatus according to claim 74, wherein the prosthesis is directly operated with the kinetic energy, as the energy transforming device transforms the electric energy into the kinetic energy.

76. An apparatus according to any of the preceding claims, wherein the prosthesis is non-inflatable.

77. An apparatus according to claim 2, wherein the control device controls the external source of energy to release energy for a determined time period.

78. An apparatus according to claim 2, wherein the control device controls the external source of energy to release energy in a determined number of energy pulses.

79. An apparatus according to any of the preceding claims, wherein the control device is adapted to control the rechargeable battery to release energy in a non-invasive manner.

80. An apparatus according to any of the preceding claims, wherein the control device comprises a wireless remote control for transmitting at least one wireless control signal for controlling the prosthesis.

81. An apparatus according to claim 80, wherein the remote control is capable of obtaining information on the condition of the prosthesis when the prosthesis is implanted and of controlling the prosthesis in response to the information.

82. An apparatus according to claim 80 or 81, wherein the wireless remote control comprises at least one external signal transmitter or transceiver and at least one internal signal receiver or transceiver implantable in the patient.

83. An apparatus according to claim 80 or 81, wherein the wireless remote control comprises at least one external signal receiver or transceiver and at least one internal signal transmitter or transceiver implantable in the patient.

84. An apparatus according to any of claims 80-83, wherein the remote control is capable of sending information related to the prosthesis from inside the patient's body to the outside thereof.

85. An apparatus according to claim 84, wherein the remote control controls the prosthesis in response to the information.

86. An apparatus according to any of claims 80-85, wherein the remote control transmits a carrier signal for carrying the control signal.

87. An apparatus according to claim 86, wherein the carrier signal is frequency, amplitude or frequency and amplitude modulated.

88. An apparatus according to claim 86 or 87, wherein the carrier signal is digital, analog or digital and analog.

89. An apparatus according to any of claims 86-88, wherein the control signal used with the carrier signal is frequency, amplitude or frequency and amplitude modulated.

90. An apparatus according to any of claims 80-89, wherein the control signal comprises a wave signal comprising one of a sound wave signal including an ultrasound wave signal, an electromagnetic wave signal including an infrared light signal, a visible light signal, an ultra violet light signal and a laser light signal, a micro wave signal, a radio wave signal, an x-ray radiation signal, and a gamma radiation signal.

91. An apparatus according to claim 80-89, wherein the control signal comprises an electric, a magnetic or an electric and magnetic field.

92. An apparatus according to any of claims 80-90, wherein the control signal is digital, analog or digital and analog.

93. An apparatus according to claim 92, wherein the remote control transmits an electromagnetic carrier wave signal for carrying the digital or analog control signal.

94. An apparatus according to any of claims 80-93, wherein the control signal is transmitted in pulses by the wireless remote control.

95. An apparatus according to any of claims 2,16,17,72-74, further comprising an implantable stabiliser for stabilising the energy released by the control device.

96. An apparatus according to claim 95, wherein the energy released by the control device comprises electric energy and the stabiliser comprises at least one capacitor.

97. An apparatus according to claim 1, wherein the penile prosthesis is operable by the released energy in a manual, mechanical, thermal or magnetic manner.

98. An apparatus according to claim 1, wherein the penile prosthesis is operable by the released energy in a non-manual, non-mechanical, non-thermal or non-magnetic manner.

99. An apparatus according to claim 17, wherein the control device is adapted to control the external source of energy to release electric energy, and further comprising an implantable capacitor for producing the train of energy pulses from the released energy.

100. An apparatus according to claim 1, further comprising an adjustment device for adjusting the penile prosthesis, wherein the adjustment device is adapted to mechanically adjust the penile prosthesis, or adapted to hydraulically adjust the penile prosthesis by using hydraulic means which is devoid of hydraulic fluid of the kind having a viscosity that substantially increases when exposed to heat or a magnetic field.

101. An apparatus according to claim 31, wherein the operation device comprises an electrical operation device.

102. An apparatus according to claim 31, wherein the operation device is powered by magnetic energy, non-magnetic energy, electromagnetic energy, non-electromagnetic energy, kinetic energy, non-kinetic energy, thermal energy or non-thermal energy.

103. An apparatus according to claim 1, wherein the control device is activated in a manual or non-manual manner to control the source of energy to release energy.

104. An apparatus according to claim 1, further comprising implantable electrical components including at least one voltage level guard.

105. An apparatus according to claim 1, further comprising implantable electrical components including a single voltage level guard.

106. An apparatus according to claim 104 or 105, wherein the electrical components are devoid of any current detector and/or charge level detector.

107. An apparatus according to any of claims 103-106, further comprising an implantable capacitor or accumulator, wherein the charge or discharge of the capacitor or accumulator is controlled by use of the voltage level guard.

108. An apparatus according to claim 16, wherein the released energy comprises electric energy and further comprising an implantable capacitor for producing the train of energy pulses.

109. An apparatus according to claim 107 or 108, wherein the capacitor has a capacity less than 0,1 µF.

110. An apparatus according to claim 1,, further comprising an implantable motor or pump for operating the penile prosthesis, wherein the control device is adapted to control the rechargeable battery to directly power the motor or pump with the released energy.

111. An apparatus according to claim 16, wherein the wireless energy comprises electromagnetic waves excluding radio waves.

112. An apparatus according to any of claims 2,16 and 17, further comprising an implantable motor or pump for operating the penile prosthesis, wherein the control device is adapted to release wireless energy in the form of a magnetic field or electromagnetic waves for direct power of the motor or pump, as the wireless energy is being released.

113. An apparatus according to claim 112, wherein the pump is not a plunger type of pump.

114. An apparatus according to any of claims 2, 16 or 17, wherein the wireless energy comprises a signal.

115. An apparatus according to claim 2, further comprising an implantable energy transforming device for transforming wireless energy directly or indirectly into energy different than the wireless energy, for operation of the penile prosthesis.

116. An apparatus according to claim 115, wherein the energy transforming device transforms the wireless energy in the form of sound waves into electric energy for operation of the penile prosthesis.

117. An apparatus according to claim 116, wherein the energy transforming device transforms the wireless energy in the form of sound waves directly into electric energy.

118. An apparatus according to claim 116 or 117, wherein the energy transforming device comprises a capacitor.

119. An apparatus according to claim 118, wherein the capacitor is adapted to produce electric pulses from the transformed electric energy.

120. An apparatus according to any of claims 115-119, further comprising an implantable motor or pump for operating the penile prosthesis, wherein the motor or pump is powered by the transformed energy.

121. An apparatus according to claim 1, wherein the penile prosthesis is adjustable in a non-manual manner.

122. An apparatus according to any of the preceding claims, wherein the penile prosthesis is embedded in a soft or gel-like material.

123. An apparatus according to any of the preceding claims, wherein the penile prosthesis is embedded in a silicone material having hardness less than 20 Shore.

124. An apparatus according to claim 16, further comprising an activatable source of energy implantable in the patient, wherein the implantable source of energy is activated by wireless energy released from the external source of energy, to supply energy which is used in connection with the operation of the prosthesis.

## Patentansprüche

1. Eine Prothesenvorrichtung zur Behandlung männlicher sexueller Impotenz, umfassend eine betreibbare Penisprothese (4), welche in die Hohlräume der Corpora Cavernosa eines Patienten implantierbar ist, um einen erigierten Peniszustand zu bewirken, wenn die Prothese betrieben wird, und eine Steuereinrichtung (6), welche von außerhalb des Körpers des Patienten betreibbar ist, **gekennzeichnet durch** eine wiederaufladbare Batterie, welche in den Patienten implantierbar ist, wobei die Steuereinrichtung die wiederaufladbare Batterie (32) steuert, um zur Verwendung in Verbindung mit dem Betrieb der Prothese Energie freizugeben, wenn die Prothese implantiert ist.

2. Vorrichtung nach Anspruch 1, desweiteren umfassend eine externe Energiequelle, wobei die Steuereinrichtung eingerichtet ist, die externe Energiequelle zu steuern, um drahtlose Energie freizugeben, zur Verwendung in Verbindung mit dem Betrieb der Prothese.

3. Vorrichtung nach Anspruch 1 oder 2, wobei die Steuereinrichtung die Prothese steuert.

4. Vorrichtung nach Anspruch 3, wobei die Steuereinrichtung eine interne Steuereinheit umfasst, die zur Steuerung der Prothese in den Patienten implantierbar ist.

5. Vorrichtung nach Anspruch 4, wobei die interne Steuereinheit programmierbar ist.

6. Vorrichtung nach Anspruch 5, wobei die Steuereinrichtung eine externe Steuereinheit umfasst, die vorgesehen ist, außerhalb des Körpers des Patienten zu sein, wobei die interne Steuereinheit durch die externe Steuereinheit programmierbar ist.

7. Vorrichtung nach Anspruch 5, wobei die interne Steuereinheit zum zeitlichen Steuern der Prothese programmierbar ist.

8. Vorrichtung nach Anspruch 7, wobei die interne Steuereinheit die Prothese gemäß eines Aktivitätszeitplan-Programms zeitlich steuert.

9. Vorrichtung nach Anspruch 7, wobei die interne Steuereinheit einen Mikroprozessor umfasst.

10. Vorrichtung nach Anspruch 6, wobei die externe Steuereinheit die interne Steuereinheit gemäß eines Lademodus mit Daten lädt, welcher nur für einen Arzt autorisiert ist.

11. Vorrichtung nach Anspruch 6, wobei die externe Steuereinheit die interne Steuereinheit gemäß eines Arztmodus steuert, welcher nur für einen Arzt autorisiert ist.

12. Vorrichtung nach Anspruch 6, wobei die externe Steuereinheit die interne Steuereinheit gemäß eines Patientenmodus steuert, welcher für den Patienten zulässig ist.

13. Vorrichtung nach Anspruch 1, wobei die wiederaufladbare Batterie eine Lebensdauer von wenigstens 10 Jahren hat.

14. Vorrichtung nach einem der Ansprüche 1 bis 13, wobei die Steuereinrichtung die Penisprothese steuert.

15. Vorrichtung nach Anspruch 1 oder 2, desweiteren umfassend eine Batterie, welche in den Patienten implantierbar ist, zum Liefern von elektrischer Energie an implantierbare, elektrische Energie verbrauchende Komponenten der Vorrichtung.

16. Vorrichtung nach Anspruch 2, wobei die Steuereinrichtung eingerichtet ist, die externe Energiequelle zu steuern, drahtlose Energie zur direkten Verwendung in Verbindung mit dem Betrieb der Penisprothese freizugeben.

17. Vorrichtung nach Anspruch 16, wobei die Steuereinrichtung eingerichtet ist, die externe Energiequelle zu steuern, drahtlose Energie in der Form einer Energieimpulsfolge zur direkten Verwendung in Verbindung mit dem Betrieb der Penisprothese intermittierend freizugeben.

18. Vorrichtung nach einem der Ansprüche 1, 2 oder 14, desweiteren umfassend einen Schalter, welcher in den Patienten zum direkten oder indirekten Anschalten des Betriebs der Penisprothese implantierbar ist.

19. Vorrichtung nach Anspruch 18, wobei der Schalter die Energielieferung von der wiederaufladbaren Batterie direkt oder indirekt beeinflusst.

20. Vorrichtung nach Anspruch 19, wobei der Schalter zwischen einem "AUS"-Modus, in welchem die wiederaufladbare Batterie nicht in Verwendung ist, und einem "AN"-Modus umschaltet, in welchem die wiederaufladbare Batterie Energie für den Betrieb der Penisprothese liefert.

21. Vorrichtung nach Anspruch 2 und 20, wobei der Schalter durch die drahtlose Energie betrieben wird, welche von der externen Energiequelle freigegeben wird.

22. Vorrichtung nach Anspruch 21, wobei die Steuereinrichtung die externe Energiequelle steuert, um die drahtlose Energie freizugeben.

23. Vorrichtung nach einem der Ansprüche 1, 2 oder 22, wobei die Steuereinrichtung eine drahtlose Fernsteuerung umfasst.

24. Vorrichtung nach Anspruch 19, wobei die Steuereinrichtung eine drahtlose Fernsteuerung zur Steuerung der wiederaufladbaren Batterie umfasst.

25. Vorrichtung nach Anspruch 24, wobei der Schalter durch die drahtlose Energie von der externen Energiequelle betreibbar ist, um zwischen einem "AUS"-Modus, in welchem die wiederaufladbare Batterie und die Fernsteuerung nicht in Verwendung sind, und einem "Bereitschaft"-Modus umzuschalten, in welchem der Fernsteuerung erlaubt ist, die wiederaufladbare Batterie zu steuern, um Energie für den Betrieb der Penisprothese zu liefern.

26. Vorrichtung nach Anspruch 14, wobei die Steuereinrichtung eine drahtlose Fernsteuerung umfasst.

27. Vorrichtung nach Anspruch 19, desweiteren umfassend eine Energietransformationseinrichtung, welche in den Patienten zum Transformieren der drahtlosen Energie in speicherbare Energie implantierbar ist, wobei die wiederaufladbare Batterie geeignet ist, die speicherbare Energie zu speichern.

28. Vorrichtung nach Anspruch 27, wobei der Schalter von einem "AUS"-Modus, in welchem die wiederaufladbare Batterie nicht in Verwendung ist, zu einem "AN"-Modus umschaltet, in welchem die wiederaufladbare Batterie Energie für den Betrieb der Penisprothese liefert.

29. Vorrichtung nach Anspruch 28, wobei die Steuereinrichtung den Schalter steuert, um zwischen dem "AN"- und "AUS"-Modus umzuschalten.

30. Vorrichtung nach Anspruch 29, wobei die Steuereinrichtung eine drahtlose Fernsteuerung umfasst.

31. Vorrichtung nach einem der Ansprüche 1, 2 oder 18, desweiteren umfassend eine Betriebseinrichtung, welche in den Patienten zum Betreiben der Penisprothese implantierbar ist.

32. Vorrichtung gemäß Anspruch 31, wobei die Steuereinrichtung die Betriebseinrichtung steuert, um die Penisprothese zu betreiben.

33. Vorrichtung nach Anspruch 32, wobei die Betriebseinrichtung eine hydraulische Einrichtung und wenigstens ein Ventil zum Steuern einer Flüssigkeitsströmung in der hydraulischen Einrichtung umfasst.

34. Vorrichtung nach Anspruch 33, wobei die Steuereinrichtung eine drahtlose Fernsteuerung zum Steuern des Ventils umfasst.

35. Vorrichtung nach Anspruch 32, wobei die Penisprothese eine hydraulische Einrichtung umfasst und die Betriebseinrichtung ein Reservoir umfasst, welches eine Flüssigkeitskammer mit einem variablen, mit der hydraulischen Einrichtung verbundenen Volumen bildet, und die Betriebseinrichtung eingerichtet ist, Flüssigkeit von der Kammer in die hydraulische Einrichtung durch Reduktion des Volumens der Kammer auszuschütten und Flüssigkeit aus der hydraulischen Einrichtung in die Kammer durch Expansion des Volumens der Kammer zurückzuziehen.

36. Vorrichtung nach Anspruch 31 oder 32, wobei die Betriebseinrichtung einen Motor umfasst.

37. Vorrichtung nach Anspruch 36, wobei der Motor einen Umdrehungsmotor umfasst und die Steuereinrichtung den Umdrehungsmotor steuert, um sich mit einer gewünschten Anzahl von Umdrehungen zu drehen.

38. Vorrichtung nach Anspruch 36, wobei der Motor einen Linearmotor umfasst.

39. Vorrichtung nach Anspruch 36, wobei der Motor einen hydraulischen oder pneumatischen Flüssigkeitsmotor umfasst und die Steuereinrichtung den Flüssigkeitsmotor steuert.

40. Vorrichtung nach Anspruch 36, wobei der Motor einen elektrischen Motor mit elektrisch leitfähigen Teilen umfasst, die aus Kunststoff gefertigt sind.

41. Vorrichtung nach einem der Ansprüche 1, 31 oder 32, wobei die Steuereinrichtung polarisierte Energie von der Energiequelle freigibt.

42. Vorrichtung nach Anspruch 31 oder 32, wobei die Steuereinrichtung eine Polarität der freigegebenen Energie verändert, um die Operationseinrichtung umzukehren.

43. Vorrichtung nach Anspruch 31 oder 32, wobei die Betriebseinrichtung einen elektrischen Motor umfasst und die freigegebene Energie elektrische Energie umfasst.

44. Vorrichtung nach einem der Ansprüche 1, 31, 32 oder 36, wobei die Penisprothese betreibbar ist, um eine umkehrbare Funktion durchzuführen.

45. Vorrichtung nach Anspruch 44, desweiteren umfassend eine Umkehreinrichtung, die in den Patienten zum Umkehren der von der Penisprothese durchgeführten Funktion implantierbar ist.

46. Vorrichtung nach Anspruch 45, wobei die Steuereinrichtung die Umkehreinrichtung steuert, um die von der Penisprothese durchgeführte Funktion umzukehren.

47. Vorrichtung nach Anspruch 45, wobei die Umkehreinrichtung eine hydraulische Einrichtung umfasst, welche ein Ventil zum Verändern der Strömungsrichtung einer Flüssigkeit in der hydraulischen Einrichtung umfasst.

48. Vorrichtung nach Anspruch 45, wobei die Umkehreinrichtung eine mechanische Umkehreinrichtung umfasst.

49. Vorrichtung nach Anspruch 45, wobei die mechanische Umkehreinrichtung einen Schalter umfasst.

50. Vorrichtung nach Anspruch 45, wobei die Umkehreinrichtung ein Getriebe umfasst.

51. Vorrichtung nach Anspruch 45, wobei die Umkehreinrichtung einen Schalter umfasst.

52. Vorrichtung nach Anspruch 51, wobei der Schalter der Umkehreinrichtung durch die freigegebene Energie betreibbar ist.

53. Vorrichtung nach Anspruch 52, wobei die Steuereinrichtung den Betrieb des Schalters der Umkehreinrichtung durch Verändern einer Polarität der freigegebenen Energie steuert, welche zu dem Schalter geliefert wird.

54. Vorrichtung nach Anspruch 51, wobei der Schalter einen elektrischen Schalter umfasst und die wiederaufladbare Batterie elektrische Energie für den Betrieb des Schalters liefert.

55. Vorrichtung nach Anspruch 51, wobei die Betriebseinrichtung einen Motor umfasst und die Umkehreinrichtung den Motor umkehrt.

56. Vorrichtung nach einem der Ansprüche 1, 2, 31 oder 32, wobei die Penisprothese eine hydraulische Einrichtung umfasst, die desweiteren eine Betriebseinrichtung umfasst, die eingerichtet ist, eine hydraulische Flüssigkeit in die hydraulische Einrichtung zu leiten.

57. Vorrichtung nach Anspruch 56, wobei die Betriebseinrichtung einen Motor umfasst.

58. Vorrichtung nach Anspruch 56 oder 57, wobei die Betriebseinrichtung einen Flüssigkeitskanal umfasst, welcher mit der hydraulischen Einrichtung der Penisprothese verbunden ist, und ein Reservoir für Flüssigkeit, wobei das Reservoir einen Teil des Kanals bildet.

59. Vorrichtung nach Anspruch 58, wobei die hydraulische Einrichtung und der Kanal frei von jeglichem Rückschlagventil ist.

60. Vorrichtung nach Anspruch 59, wobei das Reservoir eine Flüssigkeitskammer mit einem variablen Volumen bildet und die Betriebseinrichtung eingerichtet ist, Flüssigkeit von der Kammer in die hydraulische Einrichtung der Penisprothese durch Reduktion des Volumens der Kammer auszuschütten und Flüssigkeit von der hydraulischen Einrichtung in die Kammer durch Expansion des Volumens der Kammer zurückzuziehen.

61. Vorrichtung nach einem der vorangehenden Ansprüche, desweiteren umfassend wenigstens einen implantierbaren Sensor zum Erfassen wenigstens eines physikalischen Parameters des Patienten.

62. Vorrichtung nach Anspruch 61, wobei der Sensor eingerichtet ist, eine Ejakulation oder einen Druck in der Harnröhre als physikalischen Parameter direkt oder indirekt zu erfassen.

63. Vorrichtung nach Anspruch 61 oder 62, wobei die Steuereinrichtung die Penisprothese in Reaktion auf Signale von dem Sensor steuert.

64. Vorrichtung nach Anspruch 63, wobei die Steuereinrichtung eine interne Steuereinheit umfasst, welche in den Patienten implantierbar ist, wobei die interne Steuereinheit die Penisprothese in Reaktion auf Signale von dem Sensor steuert.

65. Vorrichtung nach Anspruch 64, wobei die Steuereinrichtung eine externe Steuereinheit außerhalb des Körpers des Patienten umfasst, wobei die externe Steuereinheit die Penisprothese in Reaktion auf Signale von dem Sensor steuert.

66. Vorrichtung nach Anspruch 65, wobei die externe Steuereinheit Informationen über den physikalischen Parameter speichert, welcher durch den Sensor erfasst wird, und manuell betrieben wird, um die Penisprothese basierend auf den gespeicherten Informationen zu steuern.

67. Vorrichtung nach einem der Ansprüche 61 bis 66, desweiteren umfassend wenigstens einen implantierbaren Sender zum Senden von Informationen über den physikalischen Parameter, der durch den Sensor erfasst wird.

68. Vorrichtung nach einem der vorangehenden Ansprüche, desweiteren umfassend eine externe Datenkommunikationseinrichtung, welche außerhalb des Körpers des Patienten vorgesehen ist, und eine interne Datenkommunikationseinrichtung, welche in den Patienten zum Kommunizieren mit der externen Kommunikationseinrichtung implantierbar ist, wobei die interne Datenkommunikationseinrichtung patientenbezogene Daten zurück an die externe Datenkommunikationseinrichtung liefert oder die externe. Datenkommunikationseinrichtung Daten an die internen Datenkommunikationseinrichtung liefert.

69. Vorrichtung nach Anspruch 68, wobei die interne Datenkommunikationseinrichtung Daten liefert, welche die Penisprothese betreffen.

70. Vorrichtung nach Anspruch 68 oder 69, wobei die implantierbare Kommunikationseinrichtung Daten liefert, welche wenigstens ein physikalisches Signal des Patienten betreffen.

71. Vorrichtung nach einem der vorangehenden Ansprüche, wobei die Prothese eingerichtet ist, den Penis zu steuern, um zwischen einem schlaffen und erigierten Peniszustand zu wechseln.

72. Vorrichtung nach Anspruch 71, wobei die Prothese betreibbar ist, um den Penis zu erigieren oder ihn zu erschlaffen.

73. Vorrichtung nach Anspruch 71 oder 72, wobei die Prothese eingerichtet ist, den Penis zu steuern, um zwischen einem schlaffen und erigierten Peniszustand stufenlos zu wechseln.

74. Vorrichtung nach einem der vorangehenden Ansprüche, desweiteren umfassend eine externe Energiequelle und eine implantierbare Energietransformationseinrichtung, wobei die Steuereinrichtung eingerichtet ist, die externe Energiequelle zu steuern, um drahtlose elektrische Energie freizugeben, und die Energietransformationseinrichtung eingerichtet ist, um die elektrische Energie in kinetische Energie zum Betrieb der Prothese zu transformieren.

75. Vorrichtung nach Anspruch 74, wobei die Prothese mit der kinetischen Energie direkt betrieben wird, wenn die Energietransformationseinrichtung die elektrische Energie in kinetische Energie transformiert.

76. Vorrichtung nach einem der vorangehenden Ansprüche, wobei die Prothese nicht-aufblasbar ist.

77. Vorrichtung nach Anspruch 2, wobei die Steuereinrichtung die externe Energiequelle steuert, um Energie für eine bestimmte Zeitspanne freizugeben.

78. Vorrichtung nach Anspruch 2, wobei die Steuereinrichtung die externe Energiequelle steuert, um Energie in einer vorgegebenen Anzahl von Energieimpulsen freizugeben.

79. Vorrichtung nach einem der vorangehenden Ansprüche, wobei die Steuereinrichtung eingerichtet ist, die wiederaufladbare Batterie zu steuern, um Energie in einer nicht-invasiven Weise freizugeben.

80. Vorrichtung nach einem der vorangehenden Ansprüche, wobei die Steuereinrichtung eine drahtlose Fernsteuerung umfasst, zum Übertragen wenigstens eines drahtlosen Steuersignals zum Steuern der Prothese.

81. Vorrichtung nach Anspruch 80, wobei die Fernsteuerung geeignet ist, Informationen über den Zustand der Prothese zu erhalten, wenn die Prothese implantiert ist, und die Prothese in Reaktion auf die Informationen zu steuern.

82. Vorrichtung nach Anspruch 80 oder 81, wobei die drahtlose Fernsteuerung wenigstens einen externen Signalsender oder Sendeempfänger umfasst und wenigstens einen internen Signalempfänger oder Sendeempfänger umfasst, welcher in den Patienten implantierbar ist.

83. Vorrichtung nach Anspruch 80 oder 81, wobei die drahtlose Fernsteuerung wenigstens einen externen Signalempfänger oder Sendeempfänger umfasst und wenigstens einen internen Signalsender oder Sendeempfänger umfasst, der in den Patienten implantierbar ist.

84. Vorrichtung nach einem der Ansprüche 80 bis 83, wobei die Fernsteuerung geeignet ist, Informationen, welche die Prothese betreffen, von innerhalb des Körpers des Patienten zu dessen Äußeren zu senden.

85. Vorrichtung nach Anspruch 84, wobei die Fernsteuerung die Prothese in Reaktion auf die Informationen steuert.

86. Vorrichtung nach einem der Ansprüche 80 bis 85, wobei die Fernsteuerung ein Trägersignal zum Übertragen des Steuersignals sendet.

87. Vorrichtung nach Anspruch 86, wobei das Trägersignal frequenzmoduliert, amplitudenmoduliert oder frequenz- und amplitudenmoduliert ist.

88. Vorrichtung nach Anspruch 86 oder 87, wobei das Trägersignal digital, analog oder digital und analog ist.

89. Vorrichtung nach einem der Ansprüche 86 bis 88, wobei das mit dem Trägersignal verwendete Steuersignal frequenzmoduliert, amplitudenmoduliert oder frequenz- und amplitudenmoduliert ist.

90. Vorrichtung nach einem der Ansprüche 80 bis 89, wobei das Steuersignal ein Wellensignal umfasst, welches entweder ein ein Ultraschallwellensignal umfassendes Schallwellensignal, ein ein Infrarotlichtsignal umfassendes elektromagnetisches Wellensignal, ein sichtbares Lichtsignal, ein ultraviolettes Lichtsignal und ein Laserlichtsignal, ein Mikrowellensignal, ein Radiowellensignal, ein Röntgenstrahlungssignal oder ein Gammastrahlungssignal umfasst.

91. Vorrichtung nach einem der Ansprüche 80 bis 89, wobei das Steuersignal ein elektrisches, ein magnetisches oder eine elektrisches und magnetisches Feld umfasst.

92. Vorrichtung nach einem der Ansprüche 80 bis 90, wobei das Steuersignal digital, analog oder digital und analog ist.

93. Vorrichtung nach Anspruch 92, wobei die Fernsteuerung ein elektromagnetisches Trägerwellensignal zum Übertragen des digitalen oder analogen Steuersignals sendet.

94. Vorrichtung nach einem der Ansprüche 80 bis 93, wobei das Steuersignal von der drahtlosen Fernsteuerung in Impulsen gesendet wird.

95. Vorrichtung nach einem der Ansprüche 2,16,17 oder 72 bis 74, desweiteren umfassend einen implantierbaren Stabilisator zum Stabilisieren der durch die Steuereinrichtung freigegebenen Energie.

96. Vorrichtung nach Anspruch 95, wobei die durch die Steuereinrichtung freigegebene Energie elektrische Energie umfasst und der Stabilisator wenigstens einen Kondensator umfasst.

97. Vorrichtung nach Anspruch 1, wobei die Penisprothese durch die freigegebene Energie in einer manuellen, mechanischen, thermischen oder magnetischen Weise betreibbar ist.

98. Vorrichtung nach Anspruch 1, wobei die Penisprothese durch die freigegebene Energie in einer nicht-manuellen, nicht-mechanischen, nichtthermischen oder nicht-magnetischen Weise betreibbar ist.

99. Vorrichtung nach Anspruch 17, wobei die Steuereinrichtung eingerichtet ist, die externe Energiequelle zu steuern, um elektrische Energie freizugeben, und desweiteren umfassend einen implantierbaren Kondensator zum Erzeugen der Energieimpulsfolge aus der freigegebenen Energie.

100. Vorrichtung nach Anspruch 1, desweiteren umfassend eine Einstelleinrichtung zum Einstellen der Penisprothese, wobei die Einstelleinrichtung eingerichtet ist, die Penisprothese mechanisch einzustellen, oder eingerichtet ist, die Penisprothese unter Verwendung einer hydraulischen Einrichtung hydraulisch einzustellen, welche frei von hydraulischer Flüssigkeit einer Art ist, die einer Viskosität hat, welche sich wesentlich vergrößert, wenn sie Wärme oder einem magnetischen Feld ausgesetzt ist.

101. Vorrichtung nach Anspruch 31, wobei die Betriebseinrichtung eine elektrische Betriebseinrichtung umfasst.

102. Vorrichtung nach Anspruch 31, wobei die Betriebseinrichtung mit magnetischer Energie, nicht-magnetischer Energie, elektromagnetischer Energie, nicht-elektromagnetischer Energie, kinetischer Energie, nichtkinetischer Energie, thermischer Energie oder nicht-thermischer Energie versorgt wird.

103. Vorrichtung nach Anspruch 1, wobei die Steuereinrichtung in einer manuellen oder nicht-manuellen Weise aktiviert wird, um die Energiequelle zu steuern, um Energie freizugeben.

104. Vorrichtung nach Anspruch 1, desweiteren umfassend implantierbare elektrische Komponenten, die wenigstens einen einzelnen Spannungspegelschutz umfassen.

105. Vorrichtung nach Anspruch 1, desweiteren umfassend implantierbare elektrische Komponenten, die einen einzelnen Spannungspegelschutz umfassen.

106. Vorrichtung nach Anspruch 104 oder 105, wobei die elektrischen Komponenten frei von jeglichem Stromdetektor und/oder Ladungspegeldetektor sind.

107. Vorrichtung nach einem der Ansprüche 103 bis 106, desweiteren umfassend einen implantierbaren Kondensator oder Akkumulator, wobei das Laden oder Entladen des Kondensators oder Akkumulators durch Verwendung des Spannungspegelschutzes gesteuert wird.

108. Vorrichtung nach Anspruch 16, wobei die freigegebene Energie elektrische Energie umfasst und desweiteren umfassend einen implantierbaren Kondensator zum Erzeugen der Energieimpulsfolge.

109. Vorrichtung nach Anspruch 107 oder 108, wobei der Kondensator eine Kapazität von weniger als 0,1 µF hat.

110. Vorrichtung nach Anspruch 1, desweiteren umfassend einen implantierbaren Motor oder eine implantierbare Pumpe zum Betreiben der Penisprothese, wobei die Steuereinrichtung eingerichtet ist, die wiederaufladbare Batterie zu steuern, um den Motor oder die Pumpe direkt mit der freigegebenen Energie zu versorgen.

111. Vorrichtung nach Anspruch 16, wobei die drahtlose Energie elektromagnetische Wellen umfasst, welche Radiowellen ausschließen.

112. Vorrichtung nach einem der Ansprüche 2,16 oder 17, desweiteren umfassend einen implantierbaren Motor oder eine implantierbare Pumpe zum Betreiben der Penisprothese, wobei die Steuereinrichtung eingerichtet ist, drahtlose Energie in der Form eines magnetischen Feldes oder elektromagnetischer Wellen freizugeben, zum direkten Versorgen des Motors oder der Pumpe, wenn die drahtlose Energie freigegeben wird.

113. Vorrichtung nach Anspruch 112, wobei die Pumpe keine Pumpe vom Typ einer Kolbenpumpe ist.

114. Vorrichtung nach einem der Ansprüche 2, 16 oder 17, wobei die drahtlose Energie ein Signal umfasst.

115. Vorrichtung nach Anspruch 2, desweiteren umfassend eine implantierbare Energietransformationseinrichtung zum direkten oder indirekten Transformieren drahtloser Energie in Energie, welche von der drahtlosen Energie verschieden ist, zum Betrieb der Penisprothese.

116. Vorrichtung nach Anspruch 115, wobei die Energietransformationseinrichtung die drahtlose Energie in Form von Schallwellen in elektrische Energie zum Betrieb der Penisprothese transformiert.

117. Vorrichtung nach Anspruch 116, wobei die Energietransformationseinrichtung die drahtlose Energie in Form von Schallwellen direkt in elektrische Energie transformiert.

118. Vorrichtung nach Anspruch 116 oder 117, wobei die Energietransformationseinrichtung einen Kondensator umfasst.

119. Vorrichtung nach Anspruch 118, wobei der Kondensator eingerichtet ist, elektrische Impulse aus der transformierten elektrischen Energie zu erzeugen.

120. Vorrichtung nach einem der Ansprüche 115 bis 119, desweiteren umfassend einen implantierbaren Motor oder eine implantierbare Pumpe zum Betreiben der Penisprothese, wobei der Motor oder die Pumpe von der transformierten Energie angetrieben wird.

121. Vorrichtung nach Anspruch 1, wobei die Penisprothese in einer nicht-manuellen Weise einstellbar ist.

122. Vorrichtung nach einem der vorangehenden Ansprüche, wobei die Penisprothese in einem weichen oder gelartigen Material eingebettet ist.

123. Vorrichtung nach einem der vorangehenden Ansprüche, wobei die Penisprothese in ein Silikonmaterial mit einer Härte von weniger als 20 Shore eingebettet ist.

124. Vorrichtung nach Anspruch 16, desweiteren umfassend eine aktivierbare Energiequelle, welche in den Patienten implantierbar ist, wobei die implantierbare Energiequelle durch drahtlose Energie aktiviert wird, welche von der externen Energiequelle freigegeben wird, um Energie zu liefern, welche in Verbindung mit dem Betrieb der Prothese verwendet wird.

## Revendications

1. Appareil à prothèse pour le traitement de l'impuissance sexuelle masculine, comprenant une prothèse pénienne manoeuvrable (4) implantable dans les cavités du corps caverneux d'un patient pour donner un état d'érection pénienne, lorsque la prothèse est commandée, et un dispositif de commande (6) destiné à être manoeuvré depuis l'extérieur du corps du patient, **caractérisé par** une batterie rechargeable implantable dans le patient, le dispositif de commande assurant la commande de la batterie rechargeable (32) pour la libération d'énergie destinée à être utilisée lors du fonctionnement de la prothèse, lorsque la prothèse est implantée.

2. Appareil selon la revendication 1, comprenant en outre une source externe d'énergie, et dans lequel le dispositif de commande est destiné à commander la source externe d'énergie pour libérer de l'énergie sans fil destinée à être utilisée lors du fonctionnement de la prothèse.

3. Appareil selon la revendication 1 ou 2, dans lequel le dispositif de commande assure la commande de la prothèse.

4. Appareil selon la revendication 3, dans lequel le dispositif de commande comporte une unité interne de commande implantable dans le patient pour la commande de la prothèse.

5. Appareil selon la revendication 4, dans lequel l'unité interne de commande est programmable.

6. Appareil selon la revendication 5, dans lequel le dispositif de commande comprend une unité externe de commande destinée à se trouver à l'extérieur du corps du patient, l'unité interne de commande étant programmable par l'unité externe de commande.

7. Appareil selon la revendication 5, dans lequel l'unité interne de commande est programmable par commande de la prothèse au cours du temps.

8. Appareil selon la revendication 7, dans lequel l'unité de commande interne commande la prothèse au cours du temps en fonction d'un programme de planification d'activités.

9. Appareil selon la revendication 7, dans lequel l'unité interne de commande comprend un microprocesseur.

10. Appareil selon la revendication 6, dans lequel l'unité externe de commande charge l'unité interne de commande de données en fonction d'un mode de chargement autorisé uniquement pour un docteur.

11. Appareil selon la revendication 6, dans lequel l'unité externe de commande assure la commande de l'unité interne de commande en fonction d'un mode de docteur autorisé uniquement pour un docteur.

12. Appareil selon la revendication 6, dans lequel l'unité externe de commande assure la commande de l'unité interne de commande en fonction d'un mode de patient permis pour le patient.

13. Appareil selon la revendication 1, dans lequel la batterie rechargeable a une durée de vie d'au moins 10 ans.

14. Appareil selon l'une quelconque des revendications 1 à 13, dans lequel le dispositif de commande assure la commande de la prothèse pénienne.

15. Appareil selon la revendication 1 ou 2, comprenant en outre une batterie implantable dans le patient et destinée à transmettre de l'énergie électrique aux composants implantables consommateurs d'énergie électrique de l'appareil.

16. Appareil selon la revendication 2, dans lequel le dispositif de commande est destiné à commander la source externe d'énergie pour libérer de l'énergie sans fil destinée à être utilisée directement lors du fonctionnement de la prothèse pénienne.

17. Appareil selon la revendication 16, dans lequel le dispositif de commande est destiné à commander la source externe d'énergie pour libérer par intermittence de l'énergie sans fil sous forme d'un train d'impulsions d'énergie destiné à être utilisé directement au cours du fonctionnement de la prothèse pénienne.

18. Appareil selon l'une quelconque des revendications 1, 2 et 14, comprenant en outre un commutateur implantable dans le patient et destiné à commuter directement ou indirectement le fonctionnement de la prothèse pénienne.

19. Appareil selon la revendication 18, dans lequel le commutateur affecte directement ou indirectement la transmission d'énergie depuis la batterie rechargeable.

20. Appareil selon la revendication 19, dans lequel le commutateur commute entre un mode d'ouverture dans lequel la batterie rechargeable n'est pas utilisée, et un mode de fermeture dans lequel la batterie rechargeable transmet de l'énergie pour le fonctionnement de la prothèse pénienne.

21. Appareil selon les revendications 2 et 20, dans lequel le commutateur peut être commandé par l'énergie sans fil libérée par la source externe d'énergie.

22. Appareil selon la revendication 21, dans lequel le dispositif de commande assure la commande de la source externe d'énergie pour la libération d'énergie sans fil.

23. Appareil selon l'une quelconque des revendications 1, 2 et 22, dans lequel le dispositif de commande comporte une commande à distance sans fil.

24. Appareil selon la revendication 19, dans lequel le dispositif de commande comprend une commande à distance sans fil destinée à commander la batterie rechargeable.

25. Appareil selon la revendication 24, dans lequel le commutateur peut être en fonctionnement sous la commande de l'énergie sans fil provenant de la source externe d'énergie afin qu'il commute entre un mode d'ouverture dans lequel la batterie rechargeable et la commande à distance ne sont pas utilisées et un mode d'attente dans lequel la commande à distance peut commander la batterie rechargeable pour la transmission d'énergie de fonctionnement de la prothèse pénienne.

26. Appareil selon la revendication 14, dans lequel le dispositif de commande comporte une commande à distance sans fil.

27. Appareil selon la revendication 19, comprenant en outre un dispositif de transformation d'énergie implantable dans le patient et destiné à transformer l'énergie sans fil en énergie accumulable, et dans lequel la batterie rechargeable peut accumuler l'énergie accumulable.

28. Appareil selon la revendication 27, dans lequel le commutateur commute d'un mode d'ouverture dans lequel la batterie rechargeable n'est pas utilisée à un mode de fermeture dans lequel la batterie rechargeable transmet de l'énergie de fonctionnement de la prothèse pénienne.

29. Appareil selon la revendication 28, dans lequel le dispositif de commande assurant la commande du commutateur pour la commutation entre les modes d'ouverture et de fermeture.

30. Appareil selon la revendication 29, dans lequel le dispositif de commande est une commande à distance sans fil.

31. Appareil selon l'une quelconque des revendications 1, 2 et 18, comprenant en outre un dispositif de manoeuvre implantable dans le patient et destiné à la manoeuvre de la prothèse pénienne.

32. Appareil selon la revendication 31, dans lequel le dispositif de commande assure la commande du dispositif de manoeuvre pour le fonctionnement de la prothèse pénienne.

33. Appareil selon la revendication 32, dans lequel le dispositif de manoeuvre comporte un dispositif hydraulique et au moins une soupape de commande d'un courant de fluide dans le dispositif hydraulique.

34. Appareil selon la revendication 33, dans lequel le dispositif de commande comprend une commande à distance sans fil destinée à commander la soupape.

35. Appareil selon la revendication 32, dans lequel la prothèse pénienne comprend un dispositif hydraulique et le dispositif de manoeuvre comprend un réservoir formant une chambre de fluide ayant un volume variable et raccordé au dispositif hydraulique, et le dispositif de manoeuvre est destiné à distribuer le fluide de la chambre au dispositif hydraulique par réduction du volume de la chambre et à extraire le fluide du dispositif hydraulique vers la chambre par dilatation du volume de la chambre.

36. Appareil selon la revendication 31 ou 32, dans lequel le dispositif de manoeuvre comporte un moteur.

37. Appareil selon la revendication 36, dans lequel le moteur est un moteur rotatif, et le dispositif de commande assure la commande du moteur rotatif afin qu'il tourne d'un nombre voulu de tours.

38. Appareil selon la revendication 36, dans lequel le moteur est un moteur linéaire.

39. Appareil selon la revendication 36, dans lequel le moteur est un moteur hydraulique ou pneumatique, et le dispositif de commande assure la commande de ce moteur à fluide.

40. Appareil selon la revendication 36, dans lequel le moteur est un moteur électrique ayant des parties conductrices de l'électricité formées de matière plastique.

41. Appareil selon l'une quelconque des revendications 1, 31 et 32, dans lequel le dispositif de commande libère de l'énergie polarisée depuis la source d'énergie.

42. Appareil selon la revendication 31 ou 32, dans lequel le dispositif de commande change la polarité de l'énergie libérée pour inverser le fonctionnement du dispositif de manoeuvre.

43. Appareil selon la revendication 31 ou 32, dans lequel le dispositif de manoeuvre est un moteur électrique et l'énergie libérée est de l'énergie électrique.

44. Appareil selon l'une quelconque des revendications 1, 31, 32 et 36, dans lequel la prothèse pénienne peut fonctionner en assurant une fonction réversible.

45. Appareil selon la revendication 44, comprenant en outre un dispositif d'inversion implantable dans le patient et destiné à inverser la fonction exécutée par la prothèse pénienne.

46. Appareil selon la revendication 45, dans lequel le dispositif de commande assure la commande du dispositif d'inversion pour inverser la fonction exécutée par la prothèse pénienne.

47. Appareil selon la revendication 45, dans lequel le dispositif d'inversion comprend un dispositif hydraulique comportant une soupape destinée à changer le sens de circulation d'un fluide dans le dispositif hydraulique.

48. Appareil selon la revendication 45, dans lequel le dispositif d'inversion est un dispositif d'inversion mécanique.

49. Appareil selon la revendication 45, dans lequel le dispositif d'inversion mécanique comporte un commutateur.

50. Appareil selon la revendication 45, dans lequel le dispositif d'inversion comprend une boîte à engrenage.

51. Appareil selon la revendication 45, dans lequel le dispositif d'inversion est un commutateur.

52. Appareil selon la revendication 51, dans lequel le commutateur du dispositif d'inversion peut être manoeuvré par l'énergie libérée.

53. Appareil selon la revendication 52, dans lequel le dispositif de commande assure la commande du fonctionnement du commutateur du dispositif d'inversion par changement de la polarité de l'énergie libérée transmise au commutateur.

54. Appareil selon la revendication 51, dans lequel le commutateur est un commutateur électrique et la batterie rechargeable transmet de l'énergie électrique pour le fonctionnement du commutateur;

55. Appareil selon la revendication 51, dans lequel le dispositif de manoeuvre comporte un moteur et le dispositif d'inversion provoque une inversion du fonctionnement du moteur.

56. Appareil selon l'une quelconque des revendications 1, 2, 31 et 32, dans lequel la prothèse pénienne comporte un dispositif hydraulique, et il comporte en outre un dispositif de manoeuvre destiné à conduire un fluide hydraulique dans le dispositif hydraulique.

57. Appareil selon la revendication 56, dans lequel le dispositif de manoeuvre est un moteur.

58. Appareil selon la revendication 56 ou 57, dans lequel le dispositif de manoeuvre comporte un conduit de fluide raccordé au dispositif hydraulique de la prothèse pénienne et un réservoir de fluide, le réservoir faisant partie du conduit.

59. Appareil selon la revendication 58, dans lequel le dispositif hydraulique et le conduit sont dépourvus de tout clapet de retenue.

60. Appareil selon la revendication 59, dans lequel le réservoir forme une chambre de fluide de volume variable, et le dispositif de manoeuvre est destiné à distribuer du fluide de la chambre au dispositif hydraulique de la prothèse pénienne par réduction du volume de la chambre et à extraire du fluide du dispositif hydraulique vers la chambre par dilatation du volume de la chambre.

61. Appareil selon l'une quelconque des revendications précédentes, comprenant en outre au moins un capteur implantable destiné à détecter au moins un paramètre physique du patient.

62. Appareil selon la revendication 61, dans lequel le capteur est destiné à détecter directement ou indirectement comme paramètre physique l'éjaculation ou la pression dans l'urètre.

63. Appareil selon la revendication 61 ou 62, dans lequel le dispositif de commande assure la commande de la prothèse pénienne en fonction de signaux provenant du capteur.

64. Appareil selon la revendication 63, dans lequel le dispositif de commande comprend une unité interne de commande implantable dans le patient, l'unité interne de commande assurant la commande de la prothèse pénienne en fonction de signaux provenant du capteur.

65. Appareil selon la revendication 64, dans lequel le dispositif de commande comprend une unité externe de commande placée à l'extérieur du corps du patient, l'unité externe de commande assurant la commande de la prothèse pénienne en fonction de signaux provenant du capteur.

66. Appareil selon la revendication 65, dans lequel l'unité externe de commande mémorise les informations relatives au paramètre physique détecté par le capteur et est commandée manuellement pour la commande de la prothèse pénienne en fonction des informations mémorisées.

67. Appareil selon l'une quelconque des revendications 61 à 66, comprenant en outre au moins un émetteur implantable destiné à émettre des informations relatives au paramètre physique détecté par le capteur.

68. Appareil selon l'une quelconque des revendications précédentes, comprenant en outre un communicateur externe de données destiné à se trouver en dehors du corps du patient et un communicateur interne de données implantable dans le patient et destiné à communiquer avec le communicateur externe, et dans lequel le communicateur interne de données transmet des données liées au patient vers le communicateur externe de données ou le communicateur externe de données transmet des données au communicateur interne de données.

69. Appareil selon la revendication 68, dans lequel le communicateur interne de données transmet des données liées à la prothèse pénienne.

70. Appareil selon la revendication 68 ou 69, dans lequel le communicateur implantable transmet des données liées à au moins un signal physique du patient.

71. Appareil selon l'une quelconque des revendications précédentes, dans lequel la prothèse est destinée à commander le pénis afin qu'il change entre l'état flasque et l'état d'érection pénienne.

72. Appareil selon la revendication 71, dans lequel la prothèse peut fonctionner pour provoquer l'érection du pénis ou pour le rendre flasque.

73. Appareil selon la revendication 71 ou 72, dans lequel la prothèse est destinée à commander le pénis afin qu'il change de façon progressive entre l'état flasque et l'état d'érection pénienne.

74. Appareil selon l'une quelconque des revendications précédentes, comprenant en outre une source externe d'énergie et un dispositif implantable de transformation d'énergie, dans lequel le dispositif de commande est destiné à commander la source externe d'énergie pour libérer de l'énergie électrique sans fil et le dispositif de transformation d'énergie est destiné à transformer l'énergie électrique en énergie cinétique pour le fonctionnement de la prothèse.

75. Appareil selon la revendication 74, dans lequel la prothèse est directement commandée par de l'énergie cinétique, lorsque le dispositif de transformation d'énergie transforme l'énergie électrique en énergie cinétique.

76. Appareil selon l'une quelconque des revendications précédentes, dans lequel la prothèse n'est pas gonflable.

77. Appareil selon la revendication 2, dans lequel le dispositif de commande assure la commande de la source externe d'énergie pour la libération d'énergie pendant une période déterminée.

78. Appareil selon la revendication 2, dans lequel le dispositif de commande assure la commande de la source externe d'énergie pour libérer de l'énergie sous forme d'un nombre déterminé d'impulsions d'énergie.

79. Appareil selon l'une quelconque des revendications précédentes, dans lequel le dispositif de commande est destiné à commander la batterie rechargeable afin qu'elle libère de l'énergie d'une manière non traumatisante.

80. Appareil selon l'une quelconque des revendications précédentes, dans lequel le dispositif de commande comporte une commande à distance sans fil destinée à transmettre au moins un signal de commande sans fil destiné à commander la prothèse.

81. Appareil selon la revendication 80, dans lequel la commande à distance permet l'obtention d'informations sur l'état de la prothèse lorsque la prothèse est implantée, et la commande de la prothèse en fonction des informations.

82. Appareil selon la revendication 80 ou 81, dans lequel la commande à distance sans fil comporte au moins un émetteur ou émetteur-récepteur externe de signaux et au moins un récepteur ou émetteur-récepteur interne de signaux implantable dans le patient.

83. Appareil selon la revendication 80 ou 81, dans lequel la commande à distance sans fil comporte au moins un récepteur ou émetteur-récepteur de signaux externes et au moins un émetteur ou émetteur-récepteur de signaux internes implantables dans le patient.

84. Appareil selon l'une quelconque des revendications 80 à 83, dans lequel la commande à distance permet la transmission d'informations liées à la prothèse depuis l'intérieur du corps du patient vers l'extérieur de ce corps.

85. Appareil selon la revendication 84, dans lequel la commande à distance assure la commande de la prothèse en fonction des informations.

86. Appareil selon l'une quelconque des revendications 80 à 85, dans lequel la commande à distance transmet un signal de porteuse pour le transport du signal de commande.

87. Appareil selon la revendication 86, dans lequel un signal de porteuse est modulé en fréquence, en amplitude ou en fréquence et en amplitude.

88. Appareil selon la revendication 86 ou 87, dans lequel le signal de porteuse est numérique, analogique ou numérique et analogique.

89. Appareil selon l'une quelconque des revendications 86 à 88, dans lequel le signal de commande utilisé avec le signal de porteuse est modulé en fréquence, en amplitude ou en fréquence et en amplitude.

90. Appareil selon l'une quelconque des revendications 81 à 89, dans lequel le signal de commande comporte un signal ondulatoire comprenant un signal parmi un signal d'onde acoustique comprenant un signal ultrasonore, un signal d'onde électromagnétique comprenant un signal infrarouge, un signal visible, un signal ultraviolet et un signal laser, un signal en hyperfréquences, un signal à hautes fréquences, un signal de rayons X et un signal de rayonnement gamma.

91. Appareil selon l'une des revendications 80 à 89, dans lequel le signal de commande comporte un champ électrique, un champ magnétique ou un champ électrique et magnétique.

92. Appareil selon l'une quelconque des revendications 80 à 90, dans lequel le signal de commande est numérique, analogique ou numérique et analogique.

93. Appareil selon la revendication 92, dans lequel la commande à distance émet un signal d'onde porteuse électromagnétique destiné au transport du signal numérique ou analogique de commande.

94. Appareil selon l'une quelconque des revendications 80 à 93, dans lequel le signal de commande est transmis sous forme d'impulsions par la commande à distance sans fil.

95. Appareil selon l'une quelconque des revendications 2, 16, 17, 72 à 74, comprenant en outre un stabilisateur implantable destiné à stabiliser l'énergie libérée par le dispositif de commande.

96. Appareil selon la revendication 95, dans lequel l'énergie libérée par le dispositif de commande est de l'énergie électrique et le stabilisateur comporte au moins un condensateur.

97. Appareil selon la revendication 1, dans lequel la prothèse pénienne est destinée à fonctionner sous la commande d'énergie libérée de manière manuelle, mécanique, thermique ou magnétique.

98. Appareil selon la revendication 1, dans lequel la prothèse pénienne est destinée à fonctionner avec l'énergie libérée d'une manière non manuelle, non mécanique, non thermique ou non magnétique.

99. Appareil selon la revendication 17, dans lequel le dispositif de commande est destiné à commander la source externe d'énergie pour libérer de l'énergie électrique, et comporte en outre un condensateur implantable destiné à produire un train d'impulsions d'énergie à partir de l'énergie libérée.

100. Appareil selon la revendication 1, comprenant en outre un dispositif d'ajustement destiné à ajuster la prothèse pénienne, dans lequel le dispositif d'ajustement est destiné à ajuster mécaniquement la prothèse pénienne ou est destiné à ajuster hydrauliquement la prothèse pénienne par utilisation d'un dispositif hydraulique qui est dépourvu d'un fluide hydraulique du type ayant une viscosité qui augmente notablement lors d'une exposition à la chaleur ou à un champ magnétique.

101. Appareil selon la revendication 31, dans lequel le dispositif de manoeuvre est un dispositif électrique de manoeuvre.

102. Appareil selon la revendication 31, dans lequel le dispositif de manoeuvre est alimenté par de l'énergie magnétique, de l'énergie non magnétique, de l'énergie électromagnétique, de l'énergie non électromagnétique, de l'énergie cinétique, de l'énergie non cinétique, de l'énergie thermique ou de l'énergie non thermique.

103. Appareil selon la revendication 1, dans lequel le dispositif de commande est activé de manière manuelle ou non-manuelle pour la commande de la source d'énergie destinée à libérer l'énergie.

104. Appareil selon la revendication 1, comprenant en outre des composants électriques implantables comprenant au moins un organe protecteur en fonction d'un niveau de tension.

105. Appareil selon la revendication 1, comprenant en outre des composants électriques implantables comprenant un seul organe protecteur en fonction d'un niveau de tension.

106. Appareil selon la revendication 104 ou 105, dans lequel les composants électriques sont dépourvus de tout détecteur de courant et/ou de tout détecteur de niveau de charge.

107. Appareil selon l'une quelconque des revendications 103 à 106, comprenant en outre un accumulateur ou condensateur implantable, et dans lequel la charge ou la décharge du condensateur ou de l'accumulateur est commandée par l'utilisation de l'organe protecteur en fonction d'un niveau de tension.

108. Appareil selon la revendication 16, dans lequel l'énergie libérée est de l'énergie électrique, et il comprend en outre un condensateur implantable destiné à produire le train d'impulsions d'énergie.

109. Appareil selon la revendication 107 ou 108, dans lequel le condensateur a une capacité inférieure à 0,1 µF.

110. Appareil selon la revendication 1, comprenant en outre un moteur ou une pompe implantable destiné à commander la prothèse pénienne, et dans lequel le dispositif de commande est destiné à commander la batterie rechargeable pour alimenter directement le moteur ou la pompe en énergie libérée.

111. Appareil selon la revendication 16, dans lequel l'énergie sans fil comprend des ondes électromagnétiques excluant les ondes à hautes fréquences.

112. Appareil selon l'une quelconque des revendications 2, 16 et 17, comprenant en outre un moteur ou une pompe implantable destiné à la manoeuvre de la prothèse pénienne, dans lequel le dispositif de commande est destiné à libérer de l'énergie sans fil sous forme d'un champ magnétique ou d'ondes électromagnétiques d'alimentation directe du moteur ou de la pompe, lorsque l'énergie sans fil est libérée.

113. Appareil selon la revendication 112, dans lequel la pompe n'est pas une pompe du type à plongeur.

114. Appareil selon l'une quelconque des revendications 2, 16 et 17, dans lequel l'énergie sans fil est un signal.

115. Appareil selon la revendication 2, comprenant en outre un dispositif implantable de transformation d'énergie destiné à transformer l'énergie sans fil directement ou indirectement en énergie différente de l'énergie sans fil, pour la commande de la prothèse pénienne.

116. Appareil selon la revendication 115, dans lequel le dispositif de transformation d'énergie transforme l'énergie sans fil sous forme d'ondes acoustiques en énergie électrique destinée à la commande de la prothèse pénienne.

117. Appareil selon la revendication 116, dans lequel le dispositif de transformation d'énergie transforme l'énergie sans fil sous forme d'ondes acoustiques directement en énergie électrique.

118. Appareil selon la revendication 116 ou 117, dans lequel le dispositif de transformation d'énergie est un condensateur.

119. Appareil selon la revendication 118, dans lequel le condensateur est destiné à produire des impulsions électriques à partir de l'énergie électrique transformée.

120. Appareil selon l'une quelconque des revendications 115 à 119, comprenant en outre un moteur ou une pompe implantable destiné à commander la prothèse pénienne, et le moteur ou la pompe est alimenté par l'énergie transformée.

121. Appareil selon la revendication 1, dans lequel la prothèse pénienne est réglable de manière non manuelle.

122. Appareil selon l'une quelconque des revendications précédentes, dans lequel la prothèse pénienne est enrobée dans un matériau souple ou analogue à un gel.

123. Appareil selon l'une quelconque des revendications précédentes, dans lequel la prothèse pénienne est enrobée dans un matériau à base de silicone ayant une dureté Shore inférieure à 20.

124. Appareil selon la revendication 16, comprenant en outre une source d'énergie qui peut être activée et qui peut être implantée dans le patient, dans lequel la source implantable d'énergie est activée par l'énergie sans fil libérée par la source externe d'énergie pour la transmission d'énergie qui est utilisée pour le fonctionnement de la prothèse.
